(19) Europäisches Patentamt European Patent Office Office européen des brevets

(11) **EP 4 772 200 A1**

(12) **EUROPEAN PATENT APPLICATION**
published in accordance with Art. 153(4) EPC

(43) Date of publication:
**08.07.2026 Bulletin 2026/28**

(21) Application number: **24859669.4**

(22) Date of filing: **26.08.2024**

(51) International Patent Classification (IPC):
*A61L 2/18* (2026.01)    *H05H 1/24* (2006.01)
*A61L 101/10* (2006.01)

(52) Cooperative Patent Classification (CPC):
**A61L 2/18; H05H 1/24;** A61L 2101/00

(86) International application number:
**PCT/JP2024/030128**

(87) International publication number:
**WO 2025/047636 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.08.2023 JP 2023138429**

(71) Applicant: **Canon Kabushiki Kaisha**
**Tokyo 146-8501 (JP)**

(72) Inventors:
• **TAKASHIMA, Kenji**
**Tokyo 146-8501 (JP)**
• **FURUKAWA, Takumi**
**Tokyo 146-8501 (JP)**
• **ATA, Yosuke**
**Tokyo 146-8501 (JP)**
• **AOTANI, Takaharu**
**Tokyo 146-8501 (JP)**
• **NAKAZAWA, Ikuo**
**Tokyo 146-8501 (JP)**

(74) Representative: **TBK**
**Bavariaring 4-6**
**80336 München (DE)**

(54) **ACTIVE OXYGEN TREATMENT DEVICE, TREATMENT METHOD USING ACTIVE OXYGEN, AND OZONE UFB-CONTAINING LIQUID PRODUCTION DEVICE**

(57)    An active oxygen treatment device capable of more reliably treating a surface of a substance to be treated with active oxygen. An active oxygen treatment device includes: an ozone UFB-containing liquid producing section that produces an ozone UFB-containing liquid in which UFBs including ozone are contained in a liquid; a first reservoir section that stores the ozone UFB-containing liquid; an energy application device that applies energy necessary to decompose at least a part of the ozone included in the UFBs into active oxygen to the ozone UFB-containing liquid in a stored state in the first reservoir section and decomposes at least a part of the ozone into the active oxygen; and an active oxygen UFB-containing liquid supply device that supplies, to a substance to be treated, an active oxygen UFB-containing liquid that has been generated in the first reservoir section by decomposing at least a part of the ozone in the UFBs into the active oxygen through the application of the energy, the UFBs including the active oxygen being contained in a liquid in the active oxygen UFB-containing liquid.

Fig. 1

**Description**

TECHNICAL FIELD

The present disclosure is directed to an active oxygen treatment device and an active oxygen treatment method using ultrafine bubbles containing ozone. Also, the present disclosure is directed to a device of generating ultrafine bubbles containing ozone.

BACKGROUND ART

PTL 1 relates to a disinfectant generating device and a disinfectant generating method of generating a disinfectant with sufficient disinfecting and sterilization effects using a liquid containing ultrafine bubbles (hereinafter, also referred to as "UFBs") of ozone.

PTL 1 discloses a disinfectant generating device including: an ozone UFB-containing liquid producing section that produces an ozone UFB-containing liquid in which UFBs of ozone are contained in a liquid; a mist generating section that generates mist of the ozone UFB-containing liquid; and an energy irradiation section that irradiates the mist of the ozone UFB-containing liquid with energy.

Also, PTL 1 discloses a disinfectant producing method including: an ozone UFB-containing liquid preparation process of preparing an ozone UFB-containing liquid in which UFBs of ozone are contained in a liquid; a mist generating process of generating mist of the ozone UFB-containing liquid; and an energy irradiation process of irradiating the mist of the ozone UFB-containing liquid with energy.

The paragraph [0048] in PTL 1 describes that the ultrafine bubbles of ozone contained in the mist particles are collapsed, decomposition of the ozone is promoted, and hydroxy radicals are generated by irradiating the mist of the ozone UFB-containing liquid with ultraviolet rays. There is a description that a disinfectant in the form of mist containing the hydroxy radicals is supplied to a subject to disinfect or sterilize the subject by an oxidation effect of the hydroxy radicals.

Also, PTL 2 describes a method of generating an ozone-containing ultrafine bubble-containing liquid including: a process of irradiating an ultrafine bubble liquid with ultraviolet rays.

CITATION LIST

PATENT LITERATURE

[PTL 1] Japanese Patent Laid-Open No. 2022-036815
[PTL 2] Japanese Patent Laid-Open No. 2023-058216

SUMMARY OF INVENTION

TECHNICAL PROBLEM

The present inventors have studied disinfectants generated by the disinfectant generating device according to PTL 1. As a result, an effect of the disinfectants on sanitization was limited, and there was still room for improvement. According to the studies made by the present inventors, the ozone-containing ultrafine bubble liquid according to PTL 2 also had a limited sanitization effect.

At least one aspect of the present disclosure is directed to providing an active oxygen treatment device capable of more reliably treating a surface of a substance to be treated with active oxygen. Furthermore, at least one aspect of the present disclosure is directed to providing a treatment method with active oxygen capable of more reliably treating a surface of a substance to be treated with active oxygen.

SOLUTION TO PROBLEM

According to at least one aspect of the present disclosure, an active oxygen treatment device comprising:

an ozone UFB-containing liquid producing section that produces an ozone UFB-containing liquid in which ultrafine bubbles comprising ozone are comprised in a liquid;
a first reservoir section that stores the ozone UFB-containing liquid;
an energy application device that applies energy necessary to decompose at least a part of the ozone comprised in the ultrafine bubbles into active oxygen to the ozone UFB-containing liquid in a stored state in the first reservoir section and decomposes at least a part of the ozone into active oxygen; and

an active oxygen UFB-containing liquid supply device that supplies, to a substance to be treated, an active oxygen UFB-containing liquid that has been generated in the first reservoir section by decomposing at least a part of the ozone in the ultrafine bubbles into the active oxygen through the application of the energy, the ultrafine bubbles comprising active oxygen being comprised in a liquid in the active oxygen UFB-containing liquid can be provided.

Furthermore, according to at least one aspect of the present disclosure, a treatment method with active oxygen, comprising:

(1) producing an ozone UFB-containing liquid in which ultrafine bubbles comprising ozone are comprised in a liquid;

(2) storing the produced ozone UFB-containing liquid in a first reservoir section;

(3) applying energy necessary to decompose at least a part of the ozone comprised in the ultrafine bubbles into active oxygen to the ozone UFB-containing liquid in a stored state in the first reservoir section, decomposing at least part of the ozone into the active oxygen, and preparing an active oxygen UFB-containing liquid in which the ultrafine bubbles comprising the active oxygen are comprised in a liquid; and

(4) supplying the active oxygen UFB-containing liquid stored in the first reservoir section to a substance to be treated can be provided.

Furthermore, according to at least one aspect of the present disclosure, an ozone UFB-containing liquid producing device that generates an ozone UFB-containing liquid in which ultrafine bubbles comprising ozone are comprised in a liquid, the ozone UFB-containing liquid generating device comprising:

a liquid reservoir section that stores a liquid,

wherein the liquid reservoir section comprises

a UFB generating device that produces a UFB-containing liquid in which the ultrafine bubbles are comprised in the liquid stored in the liquid reservoir section, and

a plasma actuator,

the plasma actuator is provided with a first electrode and a second electrode with a dielectric body sandwiched therebetween and generates an induced flow comprising the ozone by applying a voltage between the first electrode and the second electrode, and

the plasma actuator is disposed such that the induced flow flows inside the liquid reservoir section can be provided.

ADVANTAGEOUS EFFECTS OF INVENTION

According to at least one aspect of the present disclosure, it is possible to obtain an active oxygen treatment device capable of more reliably treating a surface of a substance to be treated with active oxygen. Furthermore, according to at least one aspect of the present disclosure, it is possible to obtain a treatment method with active oxygen capable of more reliably treating a surface of a substance to be treated with active oxygen.

BRIEF DESCRIPTION OF DRAWINGS

[Fig. 1] Fig. 1 is a schematic explanatory diagram of an active oxygen treatment device according to an aspect of the present disclosure.

[Fig. 2] Figs. 2A and 2B are schematic explanatory diagrams of the active oxygen treatment device according to an aspect of the present disclosure.

[Fig. 3] Fig. 3 is a schematic explanatory diagram of the active oxygen treatment device according to an aspect of the present disclosure.

[Fig. 4] Figs. 4A and 4B are schematic diagrams illustrating a configuration of a plasma actuator.

[Fig. 5] Fig. 5 is an explanatory diagram of the plasma actuator.

[Fig. 6] Figs. 6A and 6B are schematic explanatory diagrams of the active oxygen treatment device according to an aspect of the present disclosure.

[Fig. 7] Fig. 7 is a schematic explanatory diagram of the active oxygen treatment device according to an aspect of the present disclosure.

[Fig. 8] Fig. 8 is a schematic explanatory diagram of an ozone UFB-containing liquid producing device according to an aspect of the present disclosure.

EP 4 772 200 A1

[Fig. 9] Fig. 9 is a schematic explanatory diagram of the active oxygen treatment device according to an aspect of the present disclosure.
[Fig. 10] Figs. 10A and 10B are schematic explanatory diagrams of an active oxygen treatment device in an example.
[Fig. 11] Fig. 11 is a schematic explanatory diagram of an ultraviolet lamp.
[Fig. 12] Fig. 12 is a schematic explanatory diagram of an active oxygen treatment device in Comparative Example 2.
[Fig. 13] Fig. 13 is a schematic explanatory diagram of an active oxygen treatment device in Comparative Example 3.
[Fig. 14] Fig. 14 is an ESR spectrum of an active oxygen UFB-containing liquid in Example 1.

DESCRIPTION OF EMBODIMENTS

In the present disclosure, "from XX to YY" or "XX to YY" indicating a numerical range means a numerical range including a lower limit and an upper limit that are end points unless otherwise specified. In a case where numerical ranges are described in stages, an upper limit and a lower limit of each numerical range can be combined as desired.

Furthermore, in the present disclosure, for example, description such as "at least one selected from the group consisting of XX, YY, and ZZ" means any of XX, YY, ZZ, a combination of XX and YY, a combination of XX and ZZ, a combination of YY and ZZ, or a combination of XX, YY, and ZZ.

In the present disclosure, "ultrafine bubbles" refer to minute bubbles that are included in a liquid and have diameters of less than 1 $\mu$m, particularly, diameters of about 10 nm to 500 nm. Hereinafter, the "ultrafine bubbles" may be referred to as "UFBs".

Active oxygen in the present disclosure includes, for example, free radicals, such as superoxide ($\cdot O_2^-$) generated by decomposition of ozone ($O_3$) and hydroxyradicals ($\cdot OH$).

In the present disclosure, "treatment" of a substance to be treated with active oxygen includes any treatment that can be achieved by active oxygen such as surface modification (hydrophilizing treatment) of a surface to be treated of the substance to be treated with active oxygen, sanitization, deodorization, and bleaching.

Also, "germs" as targets of "sanitization" according to the present disclosure refer to microorganisms, and the microorganisms include, in addition to fungi, bacteria, unicellular algae, viruses, protozoans, and the like, animal or plant cells (stem cells, dedifferentiated cells, and differentiated cells), cultured tissues, fusion cells obtained by genetic engineering (including hybridomas), dedifferentiated cells, and transformants (microorganisms). Examples of the viruses include norovirus, rotavirus, influenza virus, adenoviruses, coronavirus, measles virus, rubella virus, hepatitis virus, herpes virus, HIV virus, and the like. Also, examples of the bacteria include staphylococcus, escherichia coli, salmonella, pseudomonas aeruginosa, Vibrio cholerae, shigella, anthrax, mycobacterium tuberculosis, Clostridium botulinum, clostridium tetani, streptococcus, and the like. Furthermore, examples of the fungi include the trichophyton, aspergillus, candida, and the like.

The present inventors have presumed the reason why performance of a treatments such as disinfection and sterilization by the disinfectant according to PTL 1 is insufficient as follows. In other words, the disinfectant according to PTL 1 is generated by irradiating the mists of the ozone UFB-containing liquid with energy. However, it is considered that even if the ozone UFB-containing liquid in the mist form is irradiated with energy, sufficient energy is not necessarily applied to each of mist particles of the ozone UFB-containing liquid. Therefore, the decomposition of ozone included in the UFBs into hydroxyradicals is insufficient, and it is considered that a most part of the treatment effect of the disinfectant according to PTL 1 is caused by ozone. Therefore, the treatment performance of the disinfectant according to PTL 1 is considered to be limited.

As a result of further studies of the present inventors on the basis of such consideration, the present inventors have found that the ozone trapped in the UFBs can be more reliably decomposed into the active oxygen by storing the ozone UFB-containing liquid including UFBs including ozone in a reservoir section and applying energy to the ozone UFB-containing liquid in the stored state. The stored state here refers to a state where there is no liquid flowing into the reservoir section and no liquid flowing out of the reservoir section.

The ozone contained in the UFBs can be more reliably decomposed into active oxygen, and the concentration of the active oxygen UFBs can be increased, by applying the energy to the ozone UFB-containing liquid in the state where the ozone UFB-containing liquid is stored in the reservoir section. Note that it is preferable to cause the ozone UFB-containing liquid to flow by stirring or the like inside the reservoir section to such an extent that the UFBs are not excessively broken when the energy is applied to the ozone UFB-containing liquid in the stored state since it is possible to apply sufficient energy to each of the ozone UFBs.

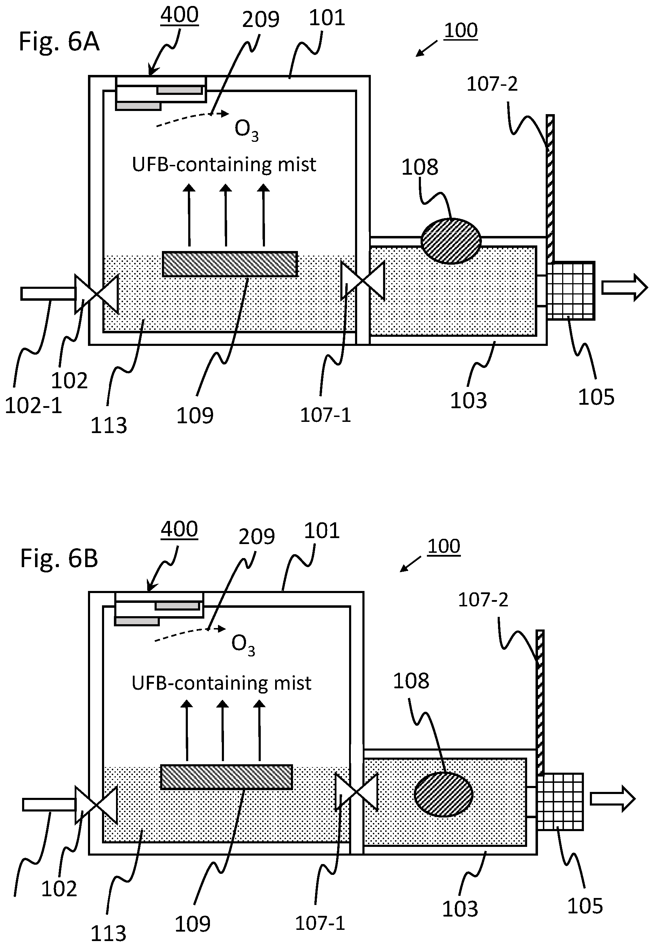

In addition, the ozone UFB liquid generating device described in the paragraphs [0037] and [0038] and Fig. 6 of PTL 2 includes a gas dissolution tank, a UFB generating unit, an ultraviolet light, a collection unit, and a reservoir unit. In the ozone UFB liquid generating device, the UFB liquid ejected from the UFB generating unit is collected by the collection unit and is delivered to the reservoir unit, and the UFB liquid stored in the reservoir unit is irradiated with ultraviolet rays. There is a description that ozone is generated and the UFB liquid inside the reservoir unit thus becomes an ozone-containing UFB liquid.

4

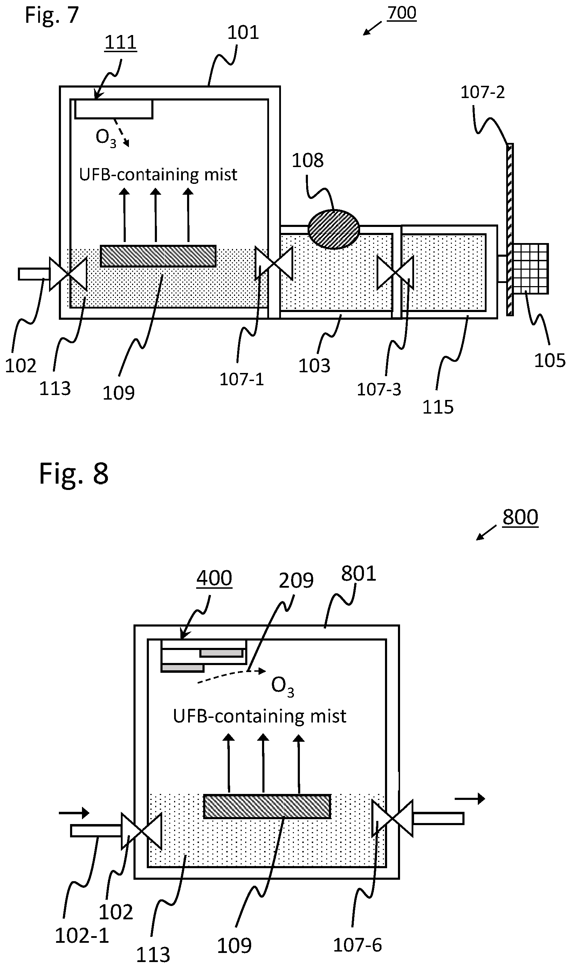

Also, the ozone UFB liquid generating device described in the paragraphs [0040] to [0042] and Fig. 7 of PTL 2 also includes a gas dissolution tank, a UFB generating unit, an ultraviolet light, a collection unit, and a reservoir unit. In the ozone UFB liquid generating device, the ultraviolet light is provided inside the collection unit, and the UFB liquid ejected from the UFB generating unit and collected by the collection unit is irradiated with ultraviolet rays by the ultraviolet light in the collection unit. The ozone-containing UFB liquid with ozone generated therein is thus delivered to the reservoir unit and is stored in the reservoir unit.

According to the studies conducted by the present inventors, the active oxygen UFB-containing liquid according to an aspect of the present disclosure was extremely superior in sanitization performance as compared with the ozone-containing UFB liquid according to PTL 2. The reason therefor is considered to be as follows.

In other words, there is a description that in an ozone UFB liquid generating method according to ,PTL 2 ultraviolet rays are absorbed by oxygen in the oxygen atmosphere and in the liquid and are dissociated into oxygen atoms by the UFB liquid being irradiated with the ultraviolet rays, the oxygen atoms are bonded to oxygen molecules to thereby generate ozone, and the UFB liquid is collected as an ozone-containing UFB liquid (see the paragraph [0018] of PTL 2). In other words, it is considered that in the method according to PTL 2, ozone is generated in the UFBs only by the irradiation with the ultraviolet rays.

On the other hand, in at least one aspect of the present disclosure, an ozone UFB-containing liquid including UFBs containing ozone in advance is prepared and energy is applied to the ozone UFB-containing liquid in a state where the ozone UFB-containing liquid is stored in the reservoir section. This is considered to enable the active oxygen UFB-containing liquid with active oxygen contained at a higher concentration in the UFBs to be generated. It is considered that as a result, the active oxygen UFB-containing liquid according to one aspect of the present disclosure can develop an increased sanitization performance as compared with the ozone-containing UFB liquid according to PTL 2.

Hereinafter, embodiments for carrying out the present disclosure will be specifically exemplified with reference to the drawings. However, dimensions, materials, shapes, relative arrangements, and the like of components described in the embodiments can be appropriately changed depending on configurations of members and various conditions to which the present disclosure is applied. In other words, the scope of the present disclosure is not intended to be limited to the following embodiments.

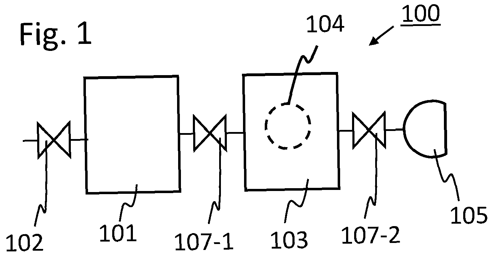

Fig. 1 is a schematic explanatory diagram of an active oxygen treatment device 100 according to at least one aspect of the present disclosure.

The active oxygen treatment device 100 includes an ozone UFB-containing liquid producing section 101 (hereinafter, also referred to as a "producing section") that produces a liquid (hereinafter, also referred to as an "ozone UFB-containing liquid") including UFBs (hereinafter, also referred to as "ozone UFBs") with ozone trapped therein. The active oxygen treatment device 100 includes an opening/closing device 102 that allows the liquid to flow from a supply source (not illustrated) of the liquid to the producing section 101 in a flow path connecting the supply source to the producing section 101 and the producing section 101 and blocks the flow of the liquid. Also, the active oxygen treatment device 100 includes a first reservoir section 103 that stores the ozone UFB-containing liquid generated by the producing section 101 and an energy application device 104 for the ozone UFB-containing liquid stored in the first reservoir section 103.

The energy application device applies energy to the ozone UFB-containing liquid in a stored state in the first reservoir section 103, decomposes at least a part of the ozone included in the UFBs into active oxygen, and generates a liquid (hereafter, also referred to as an "active oxygen UFB-containing liquid") including UFBs (hereinafter, also referred to as "active oxygen UFBs") including active oxygen therein. Also, the active oxygen treatment device 100 further includes an active oxygen UFB-containing liquid supply device (hereinafter, also simply referred to as a "supply device") 105 that supplies the active oxygen UFB-containing liquid generated in the first reservoir section 103 to a substance to be treated (not illustrated).

A flow path connecting the producing section 101 to the first reservoir section 103 is provided with an opening/closing device 107-1 that causes the liquid to flow between the producing section 101 and the first reservoir section 103 and blocks the flow of the liquid. Moreover, a flow path connecting the first reservoir section 103 to the supply device 105 is provided with an opening/closing device 107-2 that causes the liquid to flow between the first reservoir section 103 and the supply device 105 and blocks the flow of the liquid.

The opening/closing devices 107-1 and 107-2 can control the flow of a liquid into the first reservoir section 103 and the flow of the liquid out of the first reservoir section 103. Specifically, opening/closing devices 107-1 and 107-2 are configured to be able to individually stop the flowing-out of the liquid into the first reservoir section 103 and the flowing-out of the liquid from the first reservoir section 103. This enables the liquid to be in the stored state in the first reservoir section 103.

An example of a treatment method using the active oxygen treatment device illustrated in Fig. 1 will be described.

First, the opening/closing device 102 is opened to introduce the liquid from the liquid supply source into the producing section 101. Next, a liquid (UFB-containing liquid) including ultrafine bubbles is generated in presence of ozone inside the producing section 101 in a state where the opening/closing device 102 and the opening/closing device 107-1 are closed. In this manner, a liquid (ozone UFB-containing liquid) including the ozone UFBs including ozone in the UFBs is generated inside the producing section 101.

Then, the opening/closing device 107-1 is opened in a state where the opening/closing device 107-2 is closed to cause a predetermined amount of ozone UFB-containing liquid, which has been generated in the producing section 101, in the producing section 101 to move to the first reservoir section 103. Thereafter, the opening/closing device 107-1 is closed, and the ozone UFB-containing liquid is stored in the first reservoir section 103.

Next, the energy application device 104 incorporated in the first reservoir section 103 is operated for the ozone UFB-containing liquid stored in the first reservoir section 103 to decompose at least a part of ozone contained in the UFBs to obtain active oxygen, thereby causing a liquid (active oxygen-containing UFB-containing liquid) including active oxygen-containing UFBs to be generated. Here, it is important to reliably change the ozone UFBs into the active oxygen UFBs by applying sufficient energy to the ozone UFB-containing liquid stored in the reservoir section 103.

Then, the opening/closing device 107-2 is opened to supply the active oxygen UFB-containing liquid to the supply device 105, and the supply device 105 is operated to apply the active oxygen UFB-containing liquid in the reservoir section 103 to the substance to be treated, which is not illustrated. The substance to be treated to which the active oxygen-containing UFB-containing liquid has been supplied is treated with the active oxygen inside the UFBs.

Specific Configuration of Active Oxygen Treatment Apparatus

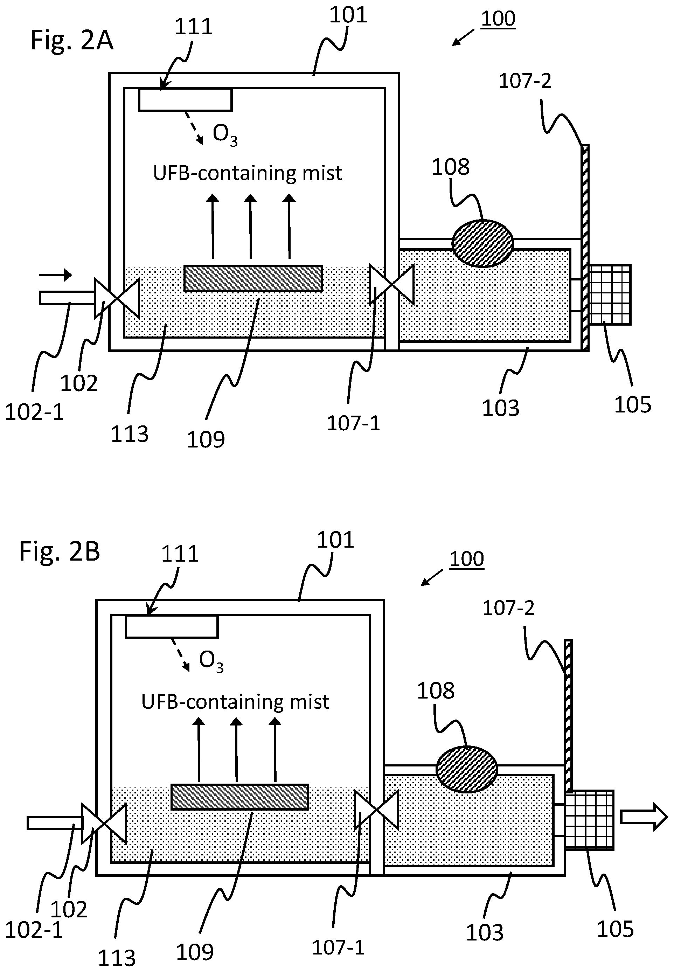

An example of a more specific configuration of the active oxygen treatment device 100 according to an aspect of the present disclosure will be described with reference to Figs. 2A and 2B.

The ozone UFB-containing liquid producing section (hereinafter, also simply referred to as a "producing section") 101 in the active oxygen treatment device 100 illustrated in Figs. 2A and 2B includes a UFB generating device 109 that generates a UFB-containing liquid from a liquid 113 such as water stored therein and an ozone generating device 111 that supplies ozone to the producing section 101. Furthermore, the active oxygen treatment device includes the opening/closing device 102 capable of blocking a supply path 102-1 of the liquid from the liquid supply source, which is not illustrated, to the inside of the producing section 101. As illustrated in Fig. 2A, the producing section 101 can store the liquid 113 therein and includes the UFB generating device 109 and the ozone generating device 111 therein.

The UFB generating device 109 is not particularly limited, and any device capable of generating UFBs in the liquid by a known way such as an ultrasonic method, a shearing method, or a pressure dissolution method may be used.

The UFB generating device 109 preferably includes an ultrasonic vibrator capable of atomizing mist containing the UFBs in the liquid to an internal space of the producing section 101. Specifically, the UFB generating device 109 can be, for example, an ultrasonic atomizer including the ultrasonic vibrator. The ultrasonic atomizer includes, for example, a substrate to be controlled, and the ultrasonic vibrator connected to the substrate by wiring. The ultrasonic vibrator is configured by a material such as an ABS resin, a silicone resin, or ceramic. As an example, an ultrasonic vibrator part made of ceramic is a disc-shaped sheet preferably with a diameter of 10 mm to 1000 mm or particularly preferably with a diameter of 10 mm to 100 mm and preferably with a thickness of 0.5 mm to 10 mm, for example, and is provided with a number of minute holes penetrating in the thickness direction and having diameters of about 1 $\mu$m to 10 $\mu$m, for example. Since the material of the ultrasonic vibrator made of ceramic has piezoelectric properties, vibration of ultrasonic waves is generated by application of a voltage.

Once ultrasonic waves are generated in a state where a rear surface of the ultrasonic vibrator is in contact with the liquid surface of the liquid, the liquid is suctioned by the minute holes, passes through the minute holes, and is then atomized as minute liquid droplets (mist) from the upper surface of the ultrasonic vibrator. In the process in which the liquid passes through the minute holes, a pressure is applied to the liquid through vibration of the ultrasonic vibrator, gas in the surroundings is taken into the liquid, and minute gas is contained in the atomized liquid droplets. In other words, the liquid droplets contain UFBs.

Although the resonance frequency of the ultrasonic vibrator is not particularly limited, the resonance frequency is preferably equal to or greater than 50 kHz or is more preferably equal to or greater than 100 kHz. Although the upper limit is also not particularly limited, the upper limit is preferably equal to or less than 3 MHz or is more preferably equal to or less than 2 MHz. A preferable range of the resonance frequency of the ultrasonic vibrator is from 50 kHz to 3 MHz, or is particularly from 100 kHz to 3 MHz. The liquid droplets to be atomized into the internal space of the producing section 101 can be formed as mist having droplet diameters of nano to micron sizes from 10 nm to 10 $\mu$m, for example, by setting the resonance frequency to be equal to or greater than 50 MHz. It is possible to extend the period of time during which the liquid can be present as liquid droplets in the internal space by forming the liquid droplets as nano-sized mist in the internal space, and it is thus possible to further increase the ozone concentration in the UFBs. Note that the mist size can be further reduced and the number concentration of the mist can be increased as the resonance frequency is increased. Although the intensity of an input to the ultrasonic vibrator is also not particularly limited, the intensity is preferably 0.1 W/cm$^2$ to 10.0 W/cm$^2$, for example. The number concentration of the mist can be increased by increasing the intensity of the input.

As the ultrasonic atomizer, a commercially available product can also be used.

Note that in a case where bubbles in the liquid droplets are relatively large, the bubbles are unstable, and gas is easily discharged to the outside of the liquid droplets. However, in a case where the bubbles in the liquid droplets are present as UFBs, the UFBs are stable and are thus atomized as UFB-containing mist while being incorporated in the liquid droplets. Additionally, the UFB-containing mist can be obtained by adjacent atomized fine liquid droplets coming into contact with each other and taking bubbles with diameters similar to their liquid droplet diameters at the time of aggregation, for example. The process of formation of the UFB-containing mist is not limited.

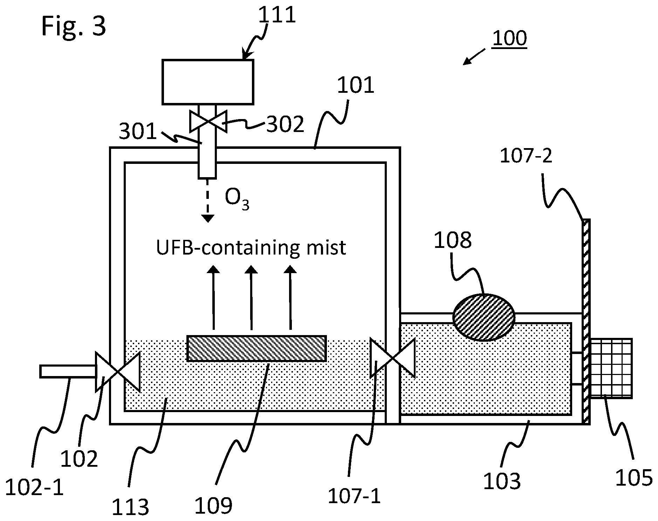

The ozone generating device 111 will be described. The ozone generating device is not particularly limited as long as it can generate ozone ($O_3$), and a commercially available ozone generating device can be used. As shown in Fig. 2A, the ozone generating device 111 may be disposed in the producing section 101 to generate ozone in the producing section 101, thereby supplying ozone into the producing section. As illustrated in Fig. 3, the ozone generating device 111 may be disposed outside the producing section 101 and may supply ozone generated outside the producing section 101 to the inside of the producing section 101. A commercially available ozone generating device may be used as the ozone generating device 111.

It is preferable to use a plasma actuator (hereinafter, also described as a "PA" in some cases) as the ozone generating device 111. The plasma actuator will be described later.

A method of obtaining the ozone UFB-containing liquid is not particularly limited. For example, it is possible to obtain the ozone UFB-containing liquid including ozone in UFBs by causing ozone generated by the ozone generating device 111 to be present in a space where the UFB generating device 109 is present inside the producing section 101, for example. Specifically, in a case where the above-described ultrasonic atomizer is used as the UFB generating device 109, for example, it is possible to produce the ozone UFB-containing liquid by causing ozone from the ozone generating device 111 to be present in the internal space of the producing section 101 and generating liquid droplets (mist) of the UFB-containing liquid in the internal space where the ozone is present. The reason therefor is considered to be because the ozone is taken into the UFBs by causing the mist of the UFB-containing liquid to be present in the space including ozone inside the producing section 101. Furthermore, on the basis of the fact that the liquid droplets are formed while the gas in the surroundings is taken into the liquid when the liquid droplets are generated from the ultrasonic vibrator as described above and the gas includes ozone, it is also considered that the UFBs in the liquid droplets generated through the operation of the ultrasonic vibrator have already included the ozone. Furthermore, the liquid droplets of the ozone UFB-containing liquid generated in the internal space of the producing section 101 are mixed with water stored in the lower portion of the producing section 101 and are repeatedly ejected as liquid droplets to the internal space by the ultrasonic atomizer, and it is thus possible to obtain the ozone UFB-containing liquid including ozone at a higher concentration.

Furthermore, examples of a particularly preferred aspect of the method of producing the ozone UFB-containing liquid include a method of disposing a plasma actuator, which will be described later, in the internal space of the producing section 101 such that an induced flow from the plasma actuator flows through the space to thereby cause liquid droplets of the UFB-containing liquid generated by the ultrasonic vibrator to be taken into the induced flow. Details will be described later.

Although the kind of liquid as a raw material for the UFB-containing liquid is not particularly limited, the liquid preferably includes water, and the liquid is more preferably water. Note that in a case where water is used, pure water or ultrapure water including no impurities, which may influence the sizes of the liquid droplets, can also be used. In addition to water, additives such as a pH adjuster and a surfactant, an organic solvent, and the like may be contained within a range in which the effects of the present disclosure are not damaged. Examples of the organic solvent include alcohols such as ethanol. In a case where water is used as the liquid, pH is preferably 1.0 to 7.0, and is more preferably 1.0 to 5.0. The UFBs are stably retained in the ozone UFB-containing liquid for a longer period of time by pH falling within the range. As a result, it is possible to further improve treatment capability with the active oxygen.

The content of UFBs with bubble diameters of equal to or less than 500 nm per unit volume in the ozone UFB-containing liquid is: preferably $1.0 \times 10^6$ to $1.0 \times 10^{10}$ bubbles/mL, is more preferably $1.0 \times 10^7$ to $1.0 \times 10^9$ bubbles/mL, and is still more preferably $1.0 \times 10^8$ to $1.0 \times 10^9$ bubbles/mL. It is possible to further improve treatment capability with the active oxygen by the content falling within the range.

The content of UFBs in the ozone UFB-containing liquid can be increased, for example, by increasing the partial pressure of ozone in a region where ozone is present in the internal space of the producing section 101 or by increasing the intensity of an input to the ultrasonic vibrator to reduce the size of the mist. The content of ultrafine bubbles can be measured by a nanoparticle size distribution measurement device utilizing laser scattering/diffraction. Specific description will be given below.

The number average bubble diameter of the UFBs in the ozone UFB-containing liquid is preferably 10 nm to 300 nm and is more preferably 20 nm to 200 nm. The number average bubble diameter falling within the range contributes to a further improvement in stability of the UFBs in the ozone UFB-containing liquid.

The number average bubble diameter of the ultrafine bubbles can be increased by using, for example, an ultrasonic vibrator with a small resonance frequency in a case where the ultrasonic vibrator is used for the production, for example. The number average bubble diameter of the UFBs included in the mist can also be reduced by using an ultrasonic vibrator with a large resonance frequency to reduce the mist size in a case where the ultrasonic vibrator is used for the production, for example. Here, a preferable resonance frequency of the ultrasonic vibrator is as described above. The number average bubble diameter of the ultrafine bubbles can be measured by a nanoparticle size distribution measurement device utilizing laser scattering and diffraction. Specific description will be given below.

pH of the ozone UFB-containing liquid is preferably 1.0 to 7.0 and is more preferably 1.0 to 5.0. It becomes easy to retain the ozone UFBs by pH falling within the range, and as a result, it is possible to further improve treatment capability with the active oxygen.

The ozone concentration in the ozone UFB-containing liquid is preferably 0.1 mg/L to 20.0 mg/L, is more preferably 0.5 mg/L to 15.0 mg/L, and is further preferably 2.0 mg/L to 15.0 mg/L. It is possible to further improve treatment capability with the active oxygen by the ozone concentration falling within the range.

As the ozone generating device 111, it is preferable to use at least a plasma actuator. The plasma actuator will be described below.

Plasma Actuator

The plasma actuator is provided with a first electrode and a second electrode sandwiching a dielectric body therebetween and can generate an induced flow including ozone by applying a voltage between the electrodes.

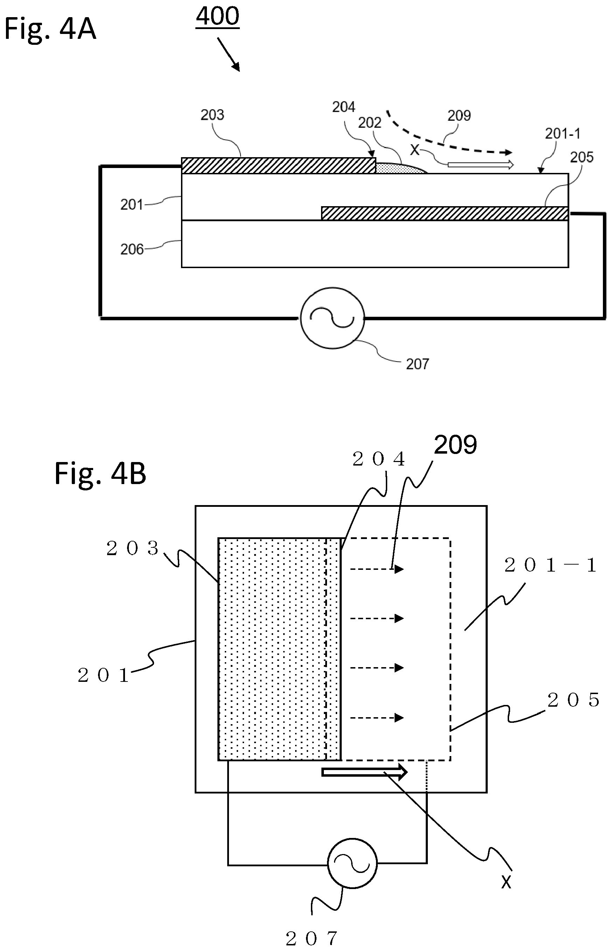

A sectional structure of an aspect of the plasma actuator 400 is illustrated in Fig. 4A. The plasma actuator is a so-called dielectric barrier discharge (DBD) plasma actuator (hereinafter, simply referred to as a "DBD-PA" in some cases) provided with a first electrode 203, which is an exposed electrode with an exposed end surface, on one surface (hereinafter, also referred to as a "first surface") of the dielectric body 201 and a second electrode 205 on a surface (hereinafter, also referred to as a "second surface") on the side opposite to the first surface. In Figs. 4A and 4B, the reference sign 206 denotes a dielectric substrate to bury the second electrode 205 in the thickness direction of the plasma actuator to prevent an induced flow from being generated from an end surface of the second electrode. A power supply 207 can apply a voltage to the first electrode and the second electrode.

In the plasma actuator 400, the first electrode 203 and the second electrode 205 disposed with the dielectric body 201 sandwiched therebetween are disposed to be shifted obliquely from each other. Dielectric barrier discharge directed from the first electrode 203 to the second electrode 205 is generated, and a jet-like flow by a surface plasma 202 is induced along the exposed portion (a part that is not covered with the first electrode) 201-1 of the first surface of the dielectric body 201 from an edge portion 204 of the first electrode 203, by applying a voltage between the first electrode 203 and the second electrode 205 (between both the electrodes) from the power supply 207. Furthermore, an air suction flow directed from the surrounding space to the electrode 203 is also generated. Electrons in the surface plasma 202 collide against oxygen molecules in the air to dissociate the oxygen molecules, thereby generating oxygen atoms. The generated oxygen atoms collide against undissociated oxygen molecules to generate ozone. Accordingly, an induced flow 209 including ozone at a high concentration is generated along the exposed portion 201-1 of the surface of the dielectric body 201 from the edge portion 204 of the first electrode 203 due to actions of the jet-like flow caused by the surface plasma 202 and the air suction flow.

In other words, the PA 400 includes the first electrode 203, the dielectric body 201, and the second electrode 205 stacked in this order, and the first electrode 203 is an exposed electrode provided on a first surface which is one surface of the dielectric body 201. The PA causes dielectric barrier discharge directed from the first electrode 203 to the second electrode 205 and injects an induced flow, which is unidirectional jet flow, in the first direction (the direction of the arrow X in Fig. 4A), which is one direction along the first surface 201-1 of the dielectric body 201, from the first electrode 203 by applying a voltage between the first electrode 203 and the second electrode 205.

Furthermore, in one section in the thickness direction of the PA, the second electrode 205 is present so as to extend in a blowing-out direction (first direction) of the induced flow. More specifically, the first electrode 203 and the second electrode 204 are disposed to be shifted obliquely from each other via the dielectric body 201 in the PA in the sectional view in the direction along the first direction of the PA, namely, the thickness direction. Also, the first electrode 203 is provided so as to cover a part of the first surface of the dielectric body 201, and at least a part of the first surface of the dielectric body 201 constitutes the exposed portion 201-1 that is not covered with the first electrode 203.

Fig. 4B is a diagram of the plasma actuator seen through the dielectric body from the side of the first surface. At least a part of the exposed portion 201-1 and the second electrode 205, which is indicated by the dashed line, overlap each other. Therefore, the overlapping is a region formed by the upper side, the lower side, and the right side of the dashed line indicating the electrode 205 in Fig. 4B and the edge portion 204.

Electrodes and Dielectric Body

Materials constituting the first electrode and the second electrode are not particularly limited as long as the materials have satisfactory conductivity. Examples of the materials that can be used include metals such as copper, aluminum, stainless steel, gold, silver, and platinum, the metals plated with or vapor-deposited with another material, conductive carbon materials such as carbon black, graphite, and carbon nanotubes, and composite materials formed by mixing such conductive carbon materials with resins or the like. The material constituting the first electrode and the material constituting the second electrode may be the same or different.

Among these, the material constituting the first electrode is preferably copper, aluminum, stainless steel, or silver from the viewpoint of avoiding corrosion of the electrode and achieving uniform discharge. For a similar reason, the material constituting the second electrode is also preferably copper, aluminum, stainless steel, or silver.

Any shapes such as flat plate shapes, wire shapes, or needle shapes may be adopted without any limitation as the shapes of the first electrode and the second electrode. The shape of the first electrode is preferably a flat plate shape. Also, the shape of the second electrode is preferably a flat plate shape. In a case where at least one of the first electrode and the second electrode has a flat plate shape, an aspect ratio (the length of the long side/the length of the short side) of the flat plate is preferably equal to or greater than two. The dielectric body is not particularly limited as long as the material has a high electrical insulating property. Examples thereof that can be used include resins such as polyimide, polyester, a fluorine resin, a silicone resin, an acrylic resin, and a phenolic resin, glass, ceramic, and composite materials obtained by mixing these with a resin or the like. Among these, ceramic and glass exhibiting excellent flame retardancy are preferably used.

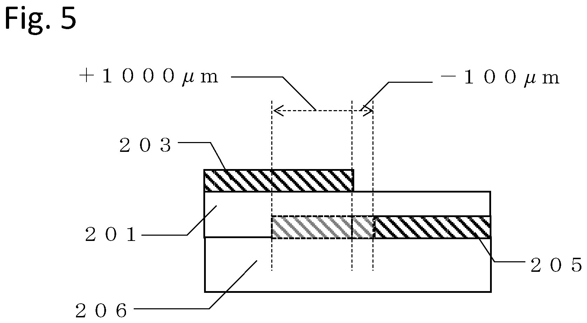

Fig. 5 is an explanatory diagram of overlapping between the first electrode 203 and the second electrode 205 in the plasma actuator 400 serving as an ozone generating device. Fig. 5 is a sectional view of the plasma actuator. When the first electrode 203 and the second electrode 205 disposed to be shifted obliquely from each other are seen from the upper side of the sectional view, an edge portion of the first electrode may be present at a formation part of the second electrode with the dielectric body sandwiched therebetween. In other words, the first electrode and the second electrode may be provided so as to overlap each other with the dielectric body sandwiched therebetween. In this case, it is preferable to prevent dielectric breakdown when a voltage is applied at the part where the first electrode and the second electrode overlap each other with the dielectric body sandwiched therebetween.

Furthermore, in a case where the first electrode and the second electrode are separated from each other when viewed from the upper side of the sectional view, it is preferable to increase the voltage in order to compensate for weakening of an electric field caused by the electrodes being separated from each other. The overlapping between the edge portion of the first electrode and the edge portion of the second electrode is more preferably -100 $\mu$m to +1000 $\mu$m when seen from the upper side of the sectional view when the length of the overlapping is assumed to be positive.

As for the thicknesses of the electrodes, there are no particular limitations for both the first electrode and the second electrode, and the thicknesses can be 10 $\mu$m to 1000 $\mu$m. If the thickness is equal to or greater than 10 $\mu$m, a resistance decreases, which promotes plasma generation. If the thickness is equal to or less than 1000 $\mu$m, electric field concentration is likely to occur, which promotes plasma generation.

As for the widths of the electrodes, there are no particular limitations for both the first electrode and the second electrode, and the widths can be equal to or greater than 1000 $\mu$m.

In a case where the edge portion of the second electrode is exposed, a plasma is also generated from the edge portion of the second electrode, and an induced flow in a direction opposite to that of the induced flow 209 derived from the first electrode may occur. In the active oxygen supply device according to this aspect, it is preferable to minimize the ozone concentration in the internal space of the active oxygen supply device other than the surface region of the substance to be treated. Furthermore, it is preferable not to generate gas flowing that disturbs the induced flow 209 inside the container. Accordingly, it is preferable not to generate an induced flow derived from the second electrode. Therefore, it is preferable that the second electrode 205 be covered with a dielectric body like the dielectric substrate 206 as illustrated in Figs. 4A and 5 or be buried in the dielectric body 201 to prevent a plasma from being generated from the edge portion of the second electrode.

The induced flow 209 including high-concentration ozone flows in the direction of a jet-like flow caused by the surface plasma along the exposed portion 201-1 of the first surface of the dielectric body 201 from the edge portion 204 of the first electrode 203, that is, the direction along the exposed portion 201-1 of the first surface of the dielectric body from the edge portion 204 of the first electrode 203. This induced flow is a gas flow having a speed of several m/s to several tens of m/s and including ozone at a high concentration. The voltage to be applied between the first electrode 203 and the second electrode 205 of the plasma actuator is not particularly limited as long as it is possible to cause the plasma actuator to generate a plasma in the aspect. Furthermore, although either a direct current voltage or an alternating current voltage may be used, an alternating current voltage is preferably used. The voltage is a pulsed voltage in a preferred aspect.

Furthermore, the amplitude and frequency of the voltage can be appropriately set in order to adjust the flow rate of the induced flow and the ozone concentration in the induced flow. In this case, the amplitude and frequency of the voltage can be appropriately selected from a viewpoint of generating an ozone concentration necessary to generate an effective active oxygen concentration or an effective active oxygen amount depending on the purpose of the treatment in the induced flow, a viewpoint of supplying the generated active oxygen to the surface region of the substance to be treated in a state where the effective active oxygen concentration or the effective active oxygen amount depending on the purpose of the treatment was maintained.

For example, the amplitude of the voltage can be 1 kV to 100 kV. Furthermore, the frequency of the voltage can be preferably equal to or greater than 1 kHz and can be more preferably 10 kHz to 100 kHz. In a case where the voltage is an alternating current voltage, although the waveform of the alternating current voltage is not particularly limited, and it is possible to adopt a sine wave, a rectangular wave, a triangular wave, or the like, it is preferable to adopt a rectangular wave from the viewpoint of quick rising of the voltage. Although the duty ratio of the voltage can also be appropriately selected, it is preferable to achieve quick rising of the voltage. Preferably, the voltage is applied such that the rising of the voltage reaching a peak from the bottom of the amplitude of the wavelength is equal to or greater than 4,000,000 V/second. Note that a value (voltage/film thickness) obtained by dividing the amplitude of the voltage to be applied between the first electrode 203 and the second electrode 205 by the film thickness of the dielectric body 201 is preferably equal to or greater than 10 kV/mm.

Production efficiency of the ozone UFB-containing liquid can be greatly improved by disposing the PA as the ozone generating device 111 in the internal space of the producing section 101 of the active oxygen treatment device according to an aspect of the present disclosure such that the induced flow from the PA flows in the internal space. The reason for this will be described below.

Figs. 6A and 6B are schematic explanatory diagrams illustrating the active oxygen treatment device according to an aspect of the present disclosure in which the plasma actuator 400 is disposed in the producing section 101 as the ozone generating device 111. In a case where the plasma actuator PA disposed in the producing section 101 is operated, the induced flow 209 generated from the PA is generated by the air in the internal space of the producing section 101 being sucked.

The mist of the UFB-containing liquid is also taken into the induced flow 209 by bringing the internal space of the producing section 101 into a state where the mist is floating by operating the ultrasonic vibrator. It is considered that as a result, the mist including the UFBs and the ozone can come into contact with each other at an extremely high probability in the induced flow 209. It is considered that as a result, the ozone is extremely efficiently taken into the UFBs in the mist. As a result, it is possible to efficiently generate the ozone UFB-containing liquid containing the ozone-containing UFBs at a high concentration.

The PA is preferably disposed such that the induced flow 209 flows inside the producing section 101. For example, the PA can be disposed as illustrated in Figs. 6A and 6B. The PA is preferably disposed such that the induced flow 209 can be circulated in the internal space of the producing section 101. Accordingly, it is preferable not to generate an induced flow from the second electrode 205.

In a case where a commercially available ozone generating device other than the PA is used as the ozone generating device 111, it is preferable to increase the ozone concentration in the producing section 101 in order to increase the probability of contact between the mist of the UFB-containing liquid and the ozone in the producing section 101. However, the probability of contact between the UFBs and the ozone is still lower than the probability of contact between the ozone and the UFBs in the induced flow even if the ozone concentration in the producing section 101 is increased, and it is considered that the amount of ozone to be actually taken into the UFBs is larger in the case where the PA is used.

On the other hand, in the case where the PA 400 serving as the ozone generating device 111 is disposed in the producing section 101, the ozone at a high concentration and the UFBs in the induced flow 209 come into contact with each other in the induced flow 209 and the ozone is efficiently taken into the UFBs by the above-described mechanism, and there is thus no need to fill the inside of the producing section 101 with ozone at a high concentration, which is advantageous in terms of an environment.

Ozone Generation Capability of PA

In the case where the PA is used as the ozone generating device, the ozone generating capability of the PA can be represented on the basis of the amount of ozone generated per unit time by Equation (A) below from the time taken until the PA is operated in a container with a predetermined volume and the ozone concentration in the container reaches a saturated state and the saturated ozone concentration (ppm) in the container. Specifically, the gas in the container under the operation of the PA is captured at regular time intervals, and the ozone concentration of the gas is measured, for example. Then, the amount of ozone generated per unit time is calculated from the time when the rate of change (rising rate) between the ozone concentration of the gas measured immediately before and the ozone concentration of the gas measured this time becomes equal to or less than 5% for the first time and the ozone concentration at that time. The measurement of the ozone concentration in the gas can be performed using, for example, an ozone detection tube. Examples of the ozone detection tube include "182SA" (trade name, manufactured by Komyo Rikagaku Kogyo K.K.).

The amount of ozone generated per unit time by the PA used as the ozone generating device in the present disclosure is preferably equal to or greater than 15 μg/min and is particularly preferably equal to or greater than 30 μg/min. Although the upper limit of the amount of generated ozone is not particularly limited, the upper limit is, for example, equal to or less than 1000 μg/min. In other words, the preferable range is from 15 μg/min to 1000 μg/min.

Equation (A)

Amount of ozone generated per unit time (μg/min)

= measured ozone concentration (ppm) × molecular weight of ozone/22.4 × 273/(273

+ room temperature (°C)) × gas volume (L) inside container

Active Oxygen UFB Producing Section 103

The ozone UFB-containing liquid generated by the ozone UFB-containing liquid producing section 101 is transferred to the first reservoir section 103 by opening the opening/closing device 107-1 and closing the opening/closing device 107-2 (Fig. 2A and the like). After a predetermined amount of ozone UFB-containing liquid in the producing section 101 is transferred to the first reservoir section 103, the opening/closing device 107-1 is closed to store the ozone UFB-containing liquid in the first reservoir section 103 (brings it into a stored state).

At least a part of ozone contained in the UFBs is decomposed into active oxygen by applying energy to the ozone UFB-containing liquid stored in the first reservoir section 103 by the energy application device 108. In this manner, an active oxygen UFB-containing liquid in which the UFBs including active oxygen are contained in the liquid is obtained in the first reservoir section 103. From the viewpoint of separately producing the ozone UFB-containing liquid and the active oxygen UFB-containing liquid to improve production efficiency of the active oxygen UFB-containing liquid, the first reservoir section 103 is preferably a tank that is different from the ozone UFB-containing liquid producing section 101 as in Fig. 2A. Also, the distance between the energy application device and the ozone UFB-containing liquid is preferably short. For example, the energy application device 108 may be provided to be able to come into contact with the ozone UFB-containing liquid in the stored state or may be disposed in the first reservoir section such that the energy application device 108 is immersed in the ozone UFB-containing liquid stored in the first reservoir section (Fig. 6B).

The energy application device 108 is not particularly limited as long as it is possible to decompose the ozone in the UFBs included in the ozone UFB-containing liquid in the stored state into active oxygen, and a known energy application device can be used. The energy application device 108 can be a heating device, an ultraviolet irradiation device, or a combination thereof, for example. The ultraviolet irradiation device includes at least an ultraviolet light source. The ultraviolet irradiation device may include the ultraviolet light source provided to be movable to uniformly apply energy to the first reservoir section.

Ultraviolet Light Source and Ultraviolet Rays

The energy application device 108 is preferably an ultraviolet irradiation device including the ultraviolet light source. Any ultraviolet light source may be adopted as long as it can apply energy necessary to decompose at least a part of the ozone included in the UFBs into active oxygen to the ozone UFB-containing liquid. In other words, the ultraviolet light source is not particularly limited as long as it can perform irradiation with ultraviolet rays to excite the ozone and generate active oxygen. Furthermore, the ultraviolet light source is not particularly limited as long as it has a wavelength and irradiance of ultraviolet rays necessary to excite ozone and obtain an effective active oxygen concentration or an effective active oxygen amount in accordance with the purpose of the treatment.

Since the peak value of the light absorption spectrum of ozone is 260 nm, for example, the peak wavelength of ultraviolet rays is preferably within a range of 200 nm to 400 nm, is more preferably in a range of 220 nm to 310 nm, is particularly preferably in a range of 253 nm to 285 nm, and is still more preferably in a range of 253 nm to 266 nm. Specific examples of the ultraviolet light source that can be used include a low-pressure mercury lamp in which mercury is sealed in quartz glass together with inert gas such as argon or neon, a cold-cathode tube ultraviolet lamp (UV-CCL), and an ultraviolet LED. The wavelength of the low-pressure mercury lamp or the cold-cathode tube ultraviolet lamp may be selected from 254 nm and the like. On the other hand, the wavelength of the ultraviolet LED is preferably selected from among 265 nm, 275 nm, 280 nm, and the like from the viewpoint of output performance.

The irradiance of the ultraviolet rays is preferably $0.2\,mW/cm^2$ to $8.0\,mW/cm^2$, is more preferably $1.5\,mW/cm^2$ to $4.0\,mW/cm^2$, and is even more preferably $1.5\,mW/cm^2$ to $3.0\,mW/cm^2$. The irradiance is a value measured by causing an irradiance detecting portion of an ultraviolet irradiance meter to be adjacent to the lamp emitting light. As the ultraviolet irradiance meter, ultraviolet irradiation amount measuring instrument (trade name: data logger ultraviolet intensity meter UV-37SD, custom-made) can be used.

Also, the amount of ultraviolet irradiation per 1 mL of ozone UFB-containing liquid is preferably 5.0 mJ/mL to 200.0 mJ/mL, is more preferably 25.0 mJ/mL to 200.0 mJ/mL, and is even more preferably 40.0 mJ/mL to 80.0 mJ/mL. The amount of irradiation can be calculated from the area of the irradiation surface of the ultraviolet lamp, the irradiation time, and the amount (liquid amount) of the ozone UFB-containing liquid to be irradiated by using a value obtained by measuring the irradiance of the ultraviolet rays as described above. Specifically, the amount of irradiation can be calculated by the irradiance of the ultraviolet rays (mW/cm$^2$) $\times$ irradiation time (sec) $\times$ area (cm$^2$) of the irradiation surface/amount (mL) of liquid adjacent to the irradiation surface.

Note that the decrease in irradiance is small within 3 cm from the irradiation surface and it is possible to regard the state as a state in contact with the irradiation surface of the ultraviolet lamp.

The time of irradiation of the ozone UFB-containing liquid with the ultraviolet rays is preferably equal to or less than 300 seconds, is particularly preferably equal to or less than 200 seconds, and is even more preferably equal to or less than 50 seconds. Although the lower limit of the irradiation time is not particularly limited, the lower limit is, for example, equal to or greater than 3 seconds, especially, equal to or greater than 5 seconds. A preferred range of irradiation time is, for example, 3 seconds to 300 seconds, especially 3 seconds to 200 seconds, and even 5 seconds to 50 seconds. It is possible to more efficiently change the ozone contained in the UFBs into active oxygen and to obtain the active oxygen UFB-containing liquid in which the UFBs including the active oxygen at a higher concentration are included by adjusting the irradiance and the area of the ultraviolet lamp to achieve the above irradiation amount within the range of the irradiation time. As a result, it is possible to more reliably perform the treatment of the substance to be treated with the active oxygen.

The content of UFBs with bubble diameters of equal to or less than 500 nm in the active oxygen UFB-containing liquid is preferably $1.0 \times 10^6$ bubbles/mL to $1.0 \times 10^{10}$ bubbles/mL, is more preferably $1.0 \times 10^7$ bubbles/mL to $1.0 \times 10^9$ bubbles/mL, and is even more preferably $1.0 \times 10^8$ bubbles/mL to $1.0 \times 10^9$ bubbles/mL. It is possible to further improve the treatment performance with the active oxygen by the content falling within the range. The content of the UFBs in the active oxygen UFB-containing liquid can be increased, for example, by increasing the amount of UFBs at the time of producing the ozone UFBs by the above-described method.

After the ozone UFB-containing liquid is brought into the stored state, and energy is applied thereto to obtain the active oxygen-containing UFB-containing liquid, the opening/closing device 107-2 is opened (Fig. 2B), and the supply device 105 is operated to apply the active oxygen-containing UFB-containing liquid in the first reservoir section 103 to the substance to be treated, which is not illustrated. The supply device 105 is not particularly limited as long as it can apply the liquid to the substance to be treated, and a known supply device can be used. The supply device 105 may be, for example, an atomizing device that atomizes the active oxygen UFB-containing liquid, a jetting device that jets the active oxygen UFB-containing liquid, or an application device that applies the active oxygen UFB-containing liquid. In the case where the active oxygen UFB-containing liquid is atomized or jetted, a known propellant such as carbon dioxide gas may be used.

The supply device 105 is preferably equipped with an atomizing device that atomizes the active oxygen UFB-containing liquid to the substance to be treated. Although it is only necessary to use a known atomizing device, the atomizing device is preferably an ultrasonic atomizer. The ultrasonic atomizer that is similar to the one described above as the UFB generating device can be used. It is possible to bring the active oxygen UFB-containing liquid into contact with the rear surface of the vibrator, to suction the liquid from minute holes, to generate mist from the surface of the vibrator, and to atomize the mist to the target.

As illustrated in Fig. 7, another aspect of the active oxygen treatment device further includes a second reservoir section. The second reservoir section is configured to be able to receive the active oxygen UFB-containing liquid generated in the first reservoir section. An active oxygen treatment device 700 includes a second reservoir section 115 capable of receiving the active oxygen UFB-containing liquid from the first reservoir section 103. An opening/closing device 107-3 enables the liquid to flow into the second reservoir section 115 from the first reservoir section 103. Also, the opening/closing devices 107-2 and 107-3 enable the liquid to be stored in the second reservoir section 115.

Moreover, the active oxygen UFB-containing liquid supply device 105 is configured to be able to supply the active oxygen UFB-containing liquid stored in the second reservoir section 115 to the substance to be treated. It is possible to select a way suitable to supply the active oxygen UFB-containing liquid to the target irrespective of the configuration of the first reservoir section, that is, the relative position with the ozone UFB-containing liquid producing section, the shape of the energy application device, and the like by including such a second reservoir section 115. It is also possible to continuously produce the active oxygen UFB-containing liquid while treating the substance to be treated by the active oxygen UFB-containing liquid supply device 105.

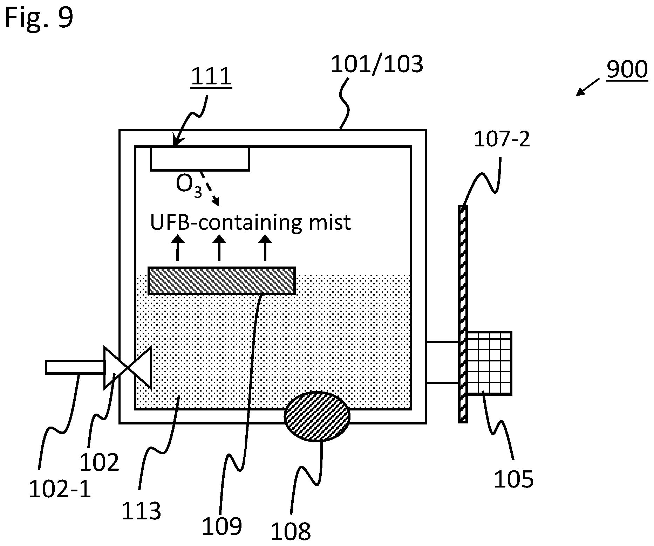

Note that in the active oxygen treatment device, the producing section 101 can also be caused to serve as the first reservoir section 103. In other words, the UFB generating device 109 and the ozone generating device 111 are disposed in the internal space of a producing section 101 in an active oxygen treatment device 900 illustrated in Fig. 9. Furthermore, the energy application device 108 for the ozone UFB-containing liquid stored in the internal space is included. The active oxygen treatment device 100 is provided with the opening/closing device 102 that causes the liquid to flow into the internal space of the producing section 101 from a liquid supply source, which is not illustrated, and blocks the flowing-in of the liquid. Also, the flow path connecting the internal space of the producing section 101 to the active oxygen UFB-containing liquid supply device 105 is provided with the opening/closing device 107-2 that enables the active oxygen UFB-containing liquid, which is to be stored in the internal space of the producing section 101, to be supplied to the supply device 105 and can block the supply.

A treatment method with active oxygen using the active oxygen treatment device 900 will be described.

First, the opening/closing device 102 is opened to introduce the liquid into the internal space of the producing section 101 in a state where the opening/closing device 107-2 is closed. Then, the ultrasonic vibrator 109 is operated in a state where the opening/closing device 102 is closed and the opening/closing device 107-2 is closed to produce the ozone UFB-containing liquid, and the ozone UFB-containing liquid 113 is stored in the internal space of the producing section 101 (also serving as the reservoir section 103). Next, the energy application device 108 is operated in a state where the opening/closing devices 102 and 107-2 are closed, that is, in a state where the ozone UFB-containing liquid is stored in the internal space of the producing section 101. In this manner, energy is applied to the ozone UFB-containing liquid stored in the internal space of the producing section 101 to decompose at least a part of the ozone included in the UFBs into active oxygen, thereby generating the active oxygen UFB-containing liquid. Then, the active oxygen UFB-containing liquid generated in the internal space of the producing section 101 is supplied to the substance to be treated (not illustrated) by the active oxygen UFB-containing liquid supply device 105, and the substance to be treated is treated with the active oxygen.

Treatment Method with Active Oxygen

At least one aspect of the present disclosure provides a treatment method with active oxygen. The treatment method with active oxygen includes the following processes (1) to (4).

(1) an ozone UFB-containing liquid production process of producing an ozone UFB-containing liquid in which UFBs including ozone are contained in a liquid

(2) a storage process of storing the produced ozone UFB-containing liquid in the first reservoir section

(3) a process of applying energy necessary to decompose at least a part of the ozone included in the ultrafine bubbles into active oxygen to the ozone UFB-containing liquid stored in the first reservoir section to decompose at least a part of the ozone into active oxygen and preparing an active oxygen UFB-containing liquid in which the ultrafine bubbles including the active oxygen are contained in a liquid

(4) an active oxygen UFB-containing liquid supply process of supplying the active oxygen UFB-containing liquid stored in the first reservoir section to a substance to be treated

The production and the storage of the ozone UFB-containing liquid, the preparation of the active oxygen UFB-containing liquid, and the supply of the active oxygen UFB-containing liquid to the substance to be treated are similar to those in the above-described active oxygen treatment device. For example, the above-described treatment method may be performed by the above-described active oxygen treatment device.

From the viewpoint of more reliably performing the treatment with active oxygen, it is preferable to apply the energy to the ozone UFB-containing liquid in the first reservoir section without the ozone UFB-containing liquid flowing into the first reservoir section and the ozone UFB-containing liquid flowing out of the first reservoir section in the process (3).

Ozone UFB-Containing Liquid Generating Device

The present disclosure also relates to an ozone UFB-containing liquid generating device that generates an ozone UFB-containing liquid. Fig. 8 illustrates an example of an ozone UFB-containing liquid generating device 800. The ozone UFB-containing liquid generating device 800 includes a liquid reservoir section 801 that stores a liquid.

The liquid reservoir section 801 includes the UFB generating device 109 that produces a UFB-containing liquid in which ultrafine bubbles are contained in a liquid 113 stored in the liquid reservoir section 801 and the plasma actuator 400. The liquid reservoir section 801 includes the opening/closing device 102 that causes the liquid 113 to flow into the liquid reservoir section 801 and blocks the flowing-in of the liquid and an opening/closing device 107-6 that can cause the ozone UFB-containing liquid to flow out of the liquid reservoir section 801. It is possible to store the liquid in the liquid reservoir section 801 by closing the opening/closing devices 102 and 107-6.

The plasma actuator is provided with the first electrode and the second electrode with the dielectric body sandwiched therebetween and generates the induced flow 209 including the ozone by applying a voltage between the first electrode and the second electrode. Also, the plasma actuator is disposed such that the induced flow 209 flows inside the liquid reservoir section 801. In the ozone UFB-containing liquid generating device 800, the ozone UFB-containing liquid is generated by the induced flow 209 coming into contact with the UFB-containing liquid.

The UFB generating device 109 and the plasma actuator 400 are similar to those described above in connection with the active oxygen treatment device. The UFB generating device 109 preferably includes an ultrasonic vibrator that causes a liquid to contain UFBs and atomizes the liquid and more preferably includes an ultrasonic atomizer. As described above, it is possible to achieve the state where liquid droplets (mist) of the UFB-containing liquid are floating inside the liquid reservoir section 801 by operating the ultrasonic vibrator. Therefore, it is easy to bring the UFB-containing liquid and the induced flow 209 into contact with each other, and it is possible to efficiently obtain the ozone UFB-containing liquid.

EXAMPLES

Although the present disclosure will be described in more detail using examples and comparative examples, aspects of the present disclosure are not limited thereto.

Example 1

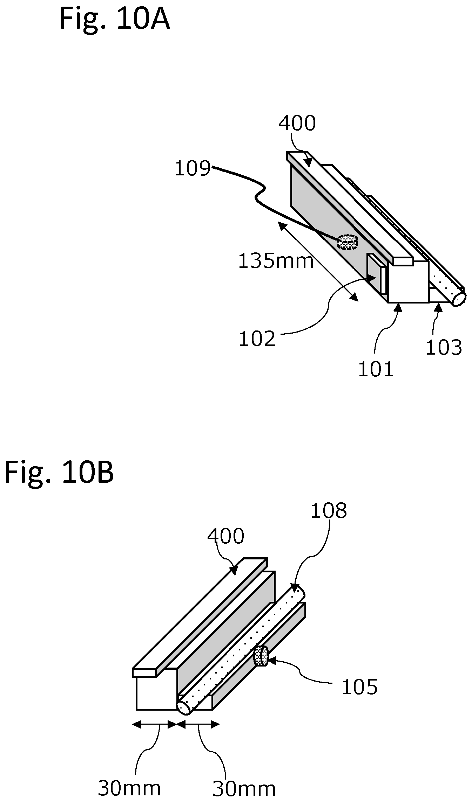

An active oxygen treatment device illustrated in Figs. 6A, 10A, and 10B was produced. Specifically, the ozone UFB-containing liquid producing section, the ozone generating device, the energy application device, an ozone UFB-containing liquid reservoir tank as the first reservoir section, and the active oxygen UFB-containing liquid supply device were provided as described below. Figs. 10A and 10B are schematic diagrams of the active oxygen treatment device as viewed from the left side and the right side, respectively.

• Ozone UFB-Containing Liquid Producing Section

The ozone UFB-containing liquid producing section 101 was produced using an ABS resin to have a size of 135 mm $\times$ 30 mm $\times$ 30 mm in height. An ultrasonic atomizer capable of storing water therein and including an ultrasonic vibrator was provided as the UFB generating device 109.

As the ultrasonic atomizer, an ultrasonic atomizer module (manufactured by REN HE) was used. The ultrasonic atomizer module included a print circuit board which was electrically controlled and an ultrasonic vibrator which was connected to the board by wiring. The ultrasonic vibrator was made of ceramic, had a circular shape with a diameter of 16 mm and a thickness of 1 mm, and had a number of minute communication holes with diameters of about 5 $\mu$m communicating the upper surface and the lower surface.

The resonance frequency of the ultrasonic vibrator was 108 kHz, and the frequency of the generated ultrasonic waves was 108 kHz. The voltage to be applied to the ultrasonic vibrator to operate the ultrasonic vibrator was an AC voltage with a frequency of 108 kHz. A voltage input as a DC voltage from the outside was converted into an AC voltage by the print circuit board, and the AC voltage at 108 kHz was input to the ultrasonic vibrator. The intensity of the input was 0.75 W/cm$^2$.

• Ozone Generating Device; Plasma Actuator

The plasma actuator 400 having the configuration illustrated in Fig. 4A was disposed inside the ozone UFB-containing liquid producing section 101 as illustrated in Fig. 6A. Specifically, the plasma actuator 400 was disposed such that the first electrode (exposed electrode) 203 was exposed to the internal space of the producing section 101 in order for the induced flow 209 generated from the PA when the PA was operated to flow through the internal space of the producing section 101. Note that the direction (depth direction) perpendicularly intersecting the paper surface of Fig. 4A is the longitudinal direction of the PA.

As the PA, a silicone film serving as the dielectric body 201 was produced to have a thickness of 100 $\mu$m by thermal pressing on a glass epoxy substrate 206 (with a length of 160 mm, a width of 10 mm, and a thickness of 1.0 mm) on which a copper foil (with a length of 150 mm, a width of 2 mm, and a thickness of 35 $\mu$m) was printed as an electrode (second electrode 205). Furthermore, a SUS plate (with a length of 135 mm and a thickness of 80 $\mu$m) was disposed as an electrode (first electrode 203) such that the longitudinal direction thereof was parallel to the longitudinal direction of the lower electrode and the distance between the closest parts of the first electrode and the second electrode was the thickness of the silicone film.

The dischargeable range was 135 mm in the longitudinal direction of the PA and the PA was disposed such that the longitudinal direction thereof coincided with the longitudinal direction (Fig. 10A) of the ozone UFB-containing liquid producing section. Setting was made such that the AC voltage of 3.0 KVpp (frequency of 75 KHz) was applied to the PA through a conversion substrate.

In the PA, the amount of generated ozone per unit time when the above AC voltage was applied was 96 $\mu$g/min. A method of measuring the amount of generated ozone was as described above.

• First Reservoir Section

A first reservoir section 103 was made of an ABS resin with a size of 135 mm in length $\times$ 16 mm in width $\times$ 8 mm in height.

• Energy Application Device Ultraviolet Lamp

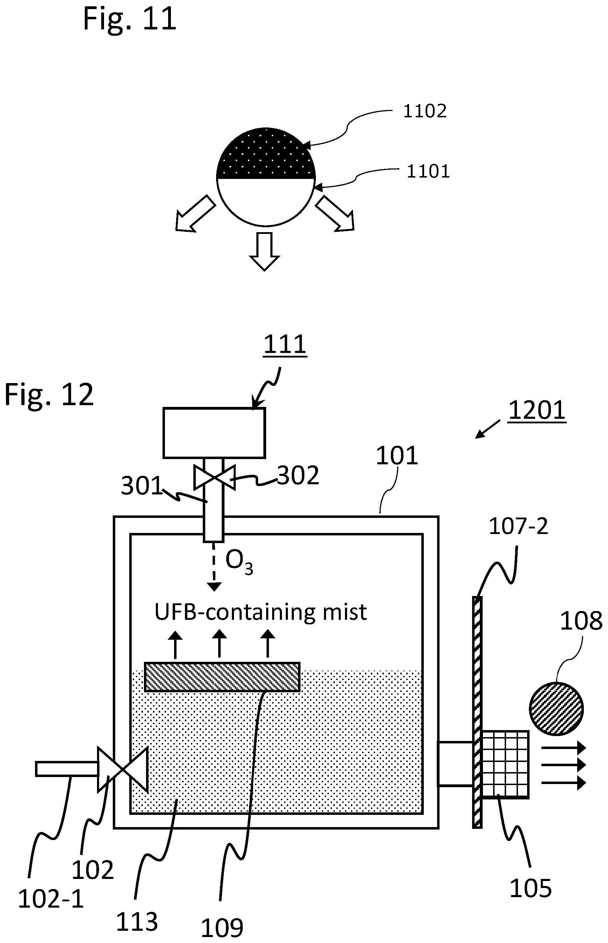

As illustrated in Fig. 6A, a tubular cold-cathode tube ultraviolet lamp (trade name: UW/9F89/9, manufactured by Stanley Electric Co., Ltd., a cylindrical shape with a diameter of 9 mm, a peak wavelength = 254 nm) was disposed as the energy application device 108 such that the internal space of the first reservoir section 103 was irradiated with ultraviolet rays. A stable ultraviolet irradiatable part of the ultraviolet lamp was a part with a length of 135 mm in the longitudinal direction of the cylindrical part, and the part was disposed to follow the longitudinal direction (the direction perpendicularly intersecting the paper surface of Fig. 6A) of the first reservoir section 103. Fig. 11 is a sectional view of the ultraviolet lamp in a direction perpendicularly intersecting the longitudinal direction. A part with a length of 135 mm in the longitudinal direction of the ultraviolet lamp was able to perform irradiation with an ultraviolet wavelength of 254 nm. The lower half of the section in Fig. 11 was an irradiatable surface 1101 capable of performing irradiation with ultraviolet rays. The upper half was a non-irradiation surface 1102 with an aluminum foil attached to the inside thereof. The area of the irradiation surface was 19.1 $cm^2$ when the ultraviolet irradiation surface faced the ozone UFB-containing liquid reservoir tank.

The ultraviolet lamp included an ultraviolet radiation part and an exterior portion of a quartz tube, and an aluminum foil was mounted on the inner half surface of the quartz tube. In other words, a semi-cylinder was the non-irradiation surface while another semi-cylinder was the irradiation surface in the circumferential direction of the ultraviolet lamp. The ultraviolet lamp was able to move in the circumferential direction in a state where the ultraviolet lamp was mounted in the ozone UFB-containing liquid reservoir tank, that is, it was possible to cause both the ultraviolet irradiation surface and the non-irradiation surface to face the inside of the ozone UFB-containing liquid reservoir tank.

The first reservoir section 103 was adjacent to the producing section 101 through an opening/closing door, and the liquid was allowed to flow in by opening the opening/closing door.

• Active Oxygen UFB-Containing Liquid Supply Device 105

An ultrasonic atomizer (manufactured by REN HE) was disposed at the center of a surface of the first reservoir section 103 on a side opposite to a surface including a connecting portion to the producing section 101. This ultrasonic atomizer was the same as the ultrasonic atomizer used in the UFB generating device 109 disposed in the internal space of the producing section 101. The configuration capable of supplying the liquid stored in the internal space of the first reservoir section 103 to the substance to be treated by the liquid coming into contact with the rear surface of the ultrasonic vibrator and by mist of the active oxygen UFB-containing liquid being generated from the surface of the ultrasonic vibrator was adopted.

Introduction of Liquid and Production of Ozone UFB-Containing Liquid

A treatment with active oxygen was performed using the thus produced active oxygen treatment device. First, the opening/closing device 102 was opened to introduce 15 mL of tap water into the internal space of the producing section 101, and the water was stored in the internal space of the producing section 101.

A direct current voltage of 20 V was applied to a power supplying conversion substrate connected to the PA, and an AC voltage of 3.0 KVpp (frequency of 75KHz) was applied between the first electrode and the second electrode of the PA to cause the PA to generate an induced flow including ozone.

Further, a direct current voltage of 5V was applied to a power applying conversion substrate to which the ultrasonic vibrator was connected in the ultrasonic atomizer serving as the UFB generating device 109 placed in the internal space of the producing section 101 to generate water mist including UFBs stored in the internal space of the producing section 101. The ultrasonic atomizer and the plasma actuator were operated for 20 minutes and were then stopped. Thereafter, the mist of the ozone UFB-containing liquid floating in the internal space of the producing section 101 was left still for 5 minutes and was settled at a lower portion of the internal space of the producing section 101. In this manner, 20 mL of ozone UFB-containing liquid was stored in the internal space of the producing section 101.

Active Oxygenation of Ozone by Ultraviolet Lamp

Then, the valve 107-1 between the producing section 101 and the first reservoir section 103 was opened, and the valve 107-2 between the first reservoir section 103 and the supply device 105 was closed to allow 15 mL of ozone UFB containing liquid to flow into the first reservoir section 103. Then, the valve 107-1 was closed to bring the ozone UFB-containing liquid into a stored state in the first reservoir section 103.

Next, the internal space of the first reservoir section, that is, the ozone UFB-containing liquid was irradiated with ultraviolet rays for 20 seconds by the irradiatable surface of the ultraviolet lamp.

For the amount of ultraviolet irradiation, a detecting section of ultraviolet irradiation amount measuring instrument (trade name: data logger ultraviolet intensity meter UV-37SD, custom-made) was brought into contact with the irradiatable surface outside the ozone UFB-containing liquid reservoir tank during warm-up operation of the ultraviolet lamp before the irradiation of the ozone UFB-containing liquid to measure the amount of ultraviolet irradiation. A numerical value stabilized at 2.0 mW/cm$^2$ was obtained immediately before the end of the warm-up operation, that is, immediately before the irradiation of the ozone UFB-containing liquid. The total amount of ultraviolet irradiation was 760 mJ, and the amount of irradiation of the ozone UFB-containing liquid was 50.7 mJ/ml.

In this way, at least a part of the ozone included in the UFBs in the ozone UFB-containing liquid was decomposed into active oxygen in the internal space of the first reservoir section 103 to thereby obtain the active oxygen UFB-containing liquid.

Treatment of Substance to Be Treated with Active Oxygen

The substance to be treated with active oxygen was placed in a direction in which the mist was ejected from the ultrasonic vibrator, which was the active oxygen UFB-containing liquid supply device. The amount of liquid to be supplied was able to be controlled by measuring the amount of atomization with a precision balance. In this example, the amount of liquid supplied as mist at a location separated by 5 cm was 3 μg per second, and the control was thus performed by an atomization time.

Evaluation 1: Measurement of Amount of Ozone Generated by Plasma Actuator per Unit Time (Capability of PA to Generate Ozone)

In order to calculate the amount of ozone generated from the PA, the PA disposed in the internal space was operated for 2 minutes under the above-described conditions for producing the ozone UFB-containing liquid before the ozone UFB-containing liquid was produced, that is, before tap water was introduced into the internal space of the producing section 101. It should be noted that the volume of the internal space of the producing section 101 was 0.122 L = 135 mm × 30 mm × 30 mm.

50 ml of gas in the internal space was sampled every 30 seconds from the start of the operation of the PA, the sampled gas was sucked into an ozone detection tube (trade name: 182SA, manufactured by Komyo Rikagaku Kogyo K.K.), and the measured ozone concentration (ppm) included in the induced flow from the plasma actuator was measured. Since the rate of change of the ozone concentration of the gas in the internal space with respect to the ozone concentration of the gas in the internal space at 30 seconds after the start of the operation of the PA became equal to or less than 5% for the first time at 1 minute after the start of the operation of the PA, it was determined that the ozone concentration of the gas in the internal space reached its saturated state at 1 minute after the start of the operation of the PA. Therefore, the amount of ozone generated per unit time by the PA was obtained by the following equation using the value of the ozone concentration in the internal space at 1 minute after the start of the operation of the PA. The room temperature was set to 25°C.

Equation (A)

Amount of ozone generated per unit time (μg/min)

= measured ozone concentration (ppm) × molecular weight of ozone of 48/22.4 × 273/(273 + room temperature (°C)) × gas volume (L) inside container

= measured ozone concentration (ppm) × 48/22.4 × 273/(273 + 25) × 0.122

Evaluation 2-1: Measurement of Ozone Concentration in Ozone UFB-Containing Liquid

For measurement of the ozone concentration in the ozone UFB-containing liquid, a pack test (trade name: Digital Pack Test and Pack Test Type WAK-O3, manufactured by Kyoritsu Chemical-check Lab., Corp), which was a colorimetric method using enzyme, was used. In the measurement method, 1.5 mL of ozone UFB-containing liquid was measured and extracted, was caused to turn purple by mixing it with dyeing powder, and the ozone concentration was calculated from the degree of color development. Samples were taken from the ozone UFB-containing liquid immediately before the energy application for active oxidation in the first reservoir section. The measurement range was 0.2 mg/L to 5mg/L, and in a case where the range was exceeded, a numerical value was obtained by performing dilution so as to achieve a measurable range, and the ozone concentration before the dilution was then calculated from the proportion of the dilution.

Evaluation 2-2: Measurement of Acidity pH of Ozone UFB-Containing Liquid

pH of the ozone UFB-containing liquid was measured by a pH meter (trade name: Twin pH meter II LAQUA twin, manufactured by As One Corporation). A value output by inputting 0.2 ml of ozone UFB-containing liquid to the device was used as a pH value.

Evaluation 2-3: Number Average Bubble Diameter of UFBs in Ozone UFB-Containing Liquid

Evaluation 3-1: Measurement of Amount of UFBs in Ozone UFB-Containing Liquid
Evaluation 3-2: Measurement of Amount of UFBs in Active Oxygen UFB-Containing Liquid

The amounts of UFBs per unit volume (bubbles/mL) in the ozone UFB-containing liquid and the active oxygen UFB-containing liquid were measured. As a measurement device, a nano-particle size distribution measuring device utilizing laser scattering/diffraction (trade name: SALD 7500, manufactured by Shimadzu Corporation) was used. In a specific measurement method, the liquid to be evaluated was input into a 5ml sample cell, and the sample cell was input into the above-described nano-particle size distribution measurement device, and the bubble sizes and the number of bubbles in the liquid in the cell were measured. Then, the number of the bubbles with bubble diameters of equal to or less than 500 nm and the number average diameter thereof (number average bubble diameter) were calculated. In this evaluation, the above-described operation was performed three times on different samples, and the arithmetic mean value thereof was regarded as the value in this evaluation. It should be noted that the measurement by the present device included noise, and in a case where the amount was less than $10^5$ bubbles/ml, it was difficult to perform separation, the amount of UFBs was not sufficient, and it was thus determined that no calculation was made.

Evaluation 4: Sanitization Test

A sanitization test for E. coli was carried out by the following procedure using the active oxygen treatment device produced in this example. All tools used in the sanitization test were subjected to high-pressure steam sterilization using an autoclave.

First, E. coli (trade name: "KWIK-STIK (Escherichia coli) ATCC8739)", manufactured by Microbiologics) was placed in a triangular flask containing an LB medium (LB medium raw material pellet, 4 tablets, total of 25 g (tradename: "LB Broth (Lennox)", manufactured by Sigma Aldrich) with 200 mL of distilled water). The triangular flask was then shaken and cultured at a temperature of 37°C for 48 hours at 80 rpm using a shaking culture machine (TA-25R-3F, manufactured by Takasaki Scientific Instruments Co., Ltd.) to obtain an E. coli liquid. The number of live bacteria in the obtained E. coli liquid was $9.2 \times 10^9$ (CFU/mL).

Control Experiment

0.01mL of the above-mentioned E. coli liquid was input into a plastic tube having the shape of a test tube with a volume of 10ml. 0.2mL of distilled water was input to the plastic tube. Then, the opening of the plastic tube was capped, and the mixture was stirred for 15 seconds using a shaking stirrer (trade name: Mini test-tube mixer MVM-10; manufactured by As One Corporation). Furthermore, 0.1 mL of sodium thiosulfate was added thereto, and the mixture was stirred for 15 seconds by the shaking stirrer.

Next, 0.2 mL of bacterial liquid in the plastic tube was then added to 9 mL of buffer liquid (phosphate buffered saline) (trade name: Gibco PBS, manufactured by Life Technologies Japan). On the basis of the resulting bacterial liquid (hereafter, also referred to as a "1/1 liquid"), the above-described buffer liquid was used to prepare a 10-time diluted bacterial liquid (1/10 diluted liquid), a 100-time diluted bacterial liquid (1/100 diluted liquid), a 1000-time diluted bacterial liquid (1/1000 diluted liquid), and a 10000-time diluted bacterial liquid (1/10000 diluted liquid). Then, 0.05 mL was taken from the 1/1 liquid, and a stamp medium (trade name: Petancheck II25 standard agar medium, manufactured by Eiken Chemical Co., Ltd.) was smeared therewith. After the placement and deployment, the stamp medium was placed in a constant temperature bath (trade name: IS600, manufactured by Yamato Scientific Co., Ltd.) and was then cultured at a temperature of 37°C for 24 hours. The number of colonies developed was counted. For the "1/10 diluted liquid", the "1/100 diluted liquid", the "1/1000 diluted liquid", and the "1/10000 diluted liquid", smearing of the stamp medium was performed, and the numbers of colonies that were developed in the medium through the culturing were counted, similarly to the above description. The results are shown in Table 1.

Table 1

|  | Number of colonies |
|---|---|
| Base liquid | ∞ |
| 1/10 diluted liquid | ∞ |
| 1/100 diluted liquid | ∞ |
| 1/1000 diluted liquid | 199 |
| 1/10000 diluted liquid | 20 |

Since a detection error is small in a case where the number of colonies falls within a range of 10 to 100, data in the above range is used as true values when the numerical values can be counted with multiple diluted liquids. From the results in Table 1, the number of colonies when the 1/10000 diluted liquid was cultured is 20, and this value is thus used. The number of E. coli present in 0.2 ml of bacterial liquid input to the buffer liquid was found to be $20 \times 10^4/0.05 \times 9.2 = 3.7 \times 10^7$ (CFU). The thus obtained number of E. coli is defined as the reference number D0 of E. coli.

Sanitization Test

0.01mL of above-described E. coli liquid was input to a plastic tube having the shape of a test tube with a volume of 10 ml. From the inlet of this plastic tube, the liquid surface of the E. coli liquid was 9.5cm away. An active oxygen treatment device was disposed at the opening of the plastic tube so that an ejecting portion of an ultrasonic vibrator serving as the active oxygen supply device 105 was in contact therewith, the opening/closing device 107-2 was opened to feed the active oxygen UFB-containing liquid stored in the first reservoir section 103 to the active oxygen supply device, the ultrasonic vibrator was operated to eject the active oxygen-containing UFB-containing liquid, and the active oxygen UFB-containing liquid was introduced into the plastic tube. Note that since the supply speed of the active oxygen UFB-containing liquid from the ultrasonic vibrator was 3 μm/second, the ejection was continued for about 33 seconds, and 0.1 mL of active oxygen UFB-containing liquid was input to the plastic tube. Thereafter, the opening of the plastic tube was capped, and stirring was performed for 15 seconds using a shaking stirrer (trade name: Mini test-tube mixer MVM-10; manufactured by As One Corporation).

Then, the plastic tube was uncapped, and 0.1 mL of sodium thiosulfate to eliminate the effects of active oxygen and ozone was added to the plastic tube, and stirring was further performed for 30 seconds. By this operation, a state where the number of E. coli did not change with further elapse of time was achieved. Then, 0.2 mL of bacterial liquid in the plastic tube was input to 9 mL of buffer liquid (phosphate buffered saline, trade name: PBS, manufactured by Life Technologies Japan). The thus obtained liquid was regarded as "treated base liquid".

Subsequently, diluted liquids were prepared by diluting the treated base liquid as a base to 10 times, 100 times, 1000 times, and 10000 times using the above-described buffer liquid. The obtained diluted liquids will also be referred to as a "1/10 diluted liquid", a "1/100 diluted liquid", a "1/1000 diluted liquid", and a "1/10000 diluted liquid", respectively.

Then, 0.05mL was collected from the treated base liquid, and the stamp medium (trade name: Petancheck II25 standard agar medium, manufactured by Eiken Chemical Co., Ltd.) was smeared with it. After the placement and deployment, the stamp medium was placed in a constant temperature bath (trade name: IS600, manufactured by Yamato Scientific Co., Ltd.) and was then cultured at a temperature of 37°C for 24 hours. The number of generated colonies was counted. For the "1/10 diluted liquid", the "1/100 diluted liquid", the "1/1000 diluted liquid", and the "1/10000 diluted liquid", smearing of the stamp medium was performed, and the numbers of colonies that were developed in the medium through the culturing were counted, similarly to the above description. The results are shown in Table 2 below.

Table 2

|  | Number of colonies |
|---|---|
| Treated base liquid | 0 |
| 1/10 diluted liquid | 0 |
| 1/100 diluted liquid | 0 |
| 1/1000 diluted liquid | 0 |
| 1/10000 diluted liquid | 0 |

As shown in the results in Table 2, although the number of colonies was 0 even in the 1/100000 diluted liquid, there was also a possibility that a countable numerical value was obtained by producing a further diluted liquid, and therefore, the results were interpreted as showing that the number was between 0 and 1 and the number of colonies was regarded as being equal to or less than 1. On the basis of this, the number D of E. coli present in 0.2 ml of bacterial liquid input to the buffer liquid was equal to or less than $1 \times 10000/0.05 \times (9 + 0.2) = 1.8 \times 10^2$ (CFU).

As a sanitization effect of the active oxygen treatment device according to this example, the ratio of change in the number D of E. coli with respect to the reference number D0 of E. coli was calculated, and -Log10 $(D/D_0)$, which was the logarithm of the change, was indicated as the decrement digit of the number of E. coli. The reference number D0 of E. coli was $3.7 \times 10^7$ as described above, the number D of E. coli was equal to or less than the evaluation limit, and therefore, the decrement digit was equal to or greater than 5.3.

Evaluation 5: Evaluation on Whether or Not Active Oxygen Has Been Generated/ESR Analysis

Electron spin resonance (ESR) analysis was performed to confirm the generation of active oxygen. ESR is a spectroscopic analysis for observing transition between levels that occur when unpaired electrons are placed in a magnetic field, and it is possible to analyze short-lived free radicals, including active oxygen such as hydroxy radicals. A specific way is to capture short-lived radicals with a spin-trap reagent to generate and detect relatively stable radical adducts, and active oxygen such as hydroxy radicals detects the radical adducts with 5,5-dimethyl-1-pyrroline-N-oxide (DMPO) as a spin-trap reagent.

In this evaluation, 90 $\mu$L of sample collected by injecting the active oxygen UFB-containing liquid for 30 seconds from the ultrasonic atomizer and 10 $\mu$L of spin-trap reagent DMPO (FUJIFILM Wako Pure Chemical Corporation) kept refrigerated at a temperature of 5°C were mixed to prepare a DMPO mixture liquid. The DMPO mixture liquid was poured into a glass capillary having an inner diameter of 1 mm. Then, the capillary with the DMPO mixture liquid poured therein was set in an electron spin resonance apparatus (ESR) (trade name: JES-X310; manufactured by JEOL Ltd.), and a spectrum was obtained. An Mn marker for data correction was also obtained at the same time. Through the ESR analysis, a spectrum (hereinafter, also referred to as an "ESR spectrum") in which the horizontal axis indicated intensity of a magnetic field and the vertical axis indicated intensity of a signal (peak) was obtained.

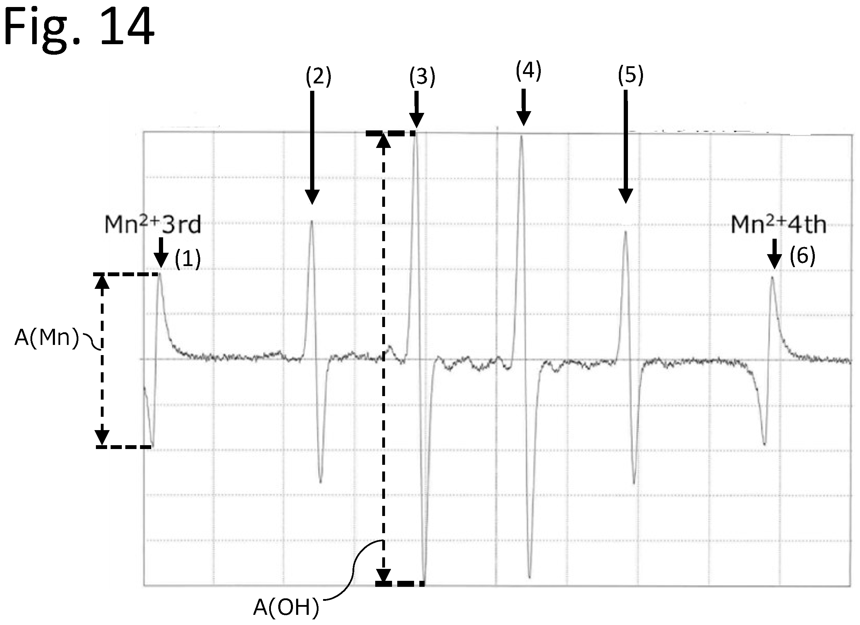

The presence of the DMPO-OH radical adduct can be confirmed by the fact that in the ESR spectrum described above, peaks of satisfactory symmetry are observed at intervals of 1.49 millitesla in the order of small, large, large and small between periodic signals, specifically, Mn marker signals. Fig. 14 illustrates an ESR spectrum of the active oxygen UFB-containing liquid which was prepared in Example 1. Peaks (1) and (6) in the ESR spectrum shown in Fig. 14 represent peaks attributable to the Mn marker, while peaks (2) to (5) represent peaks attributable to the DMPO-OH radical adduct.

The amount of OH radicals can be obtained from the peak intensity of the DMPO-OH radical adduct/the peak intensity of the Mn marker (hereinafter, also referred to as an "OH radical signal ratio"). As the peak derived from the DMPO-OH radical adduct to be used for calculating the OH radical signal ratio, the peak with the highest intensity is selected from among the peaks (2) to (5) attributable to the DMPO-OH radicals. Furthermore, as a peak derived from the Mn marker to be used for calculating the OH radical signal ratio, a peak on a low magnetic field side (the peak (1) in Fig. 14) is used. Moreover, as the peak intensity to be used for calculating the OH radical signal ratio, both amplitudes (A(Mn) and A(OH) in Fig. 14) of the peaks to be used for the calculation are used.

The value of the OH radical signal ratio is at least equal to or greater than 0.10, is preferably equal to or greater than 0.20, is more preferably equal to or greater than 0.30, is yet more preferably equal to or greater than 0.50, and is particularly preferably equal to or greater than 1.00. Although the upper limit of the OH radical signal ratio is not particularly limited, the upper limit is preferably equal to or less than 3.00, for example, or particularly, equal to or less than 2.50. The preferable numerical range of the OH radical signal ratio is from 0.10 to 3.00, from 0.20 to 3.00, from 0.30 to 3.00, from 0.50 to 3.00, or further, from 1.00 to 2.50.

It should be noted that in a case where the OH radical signal ratio was less than 0.10 and in a case where a clear peak derived from the DMPO-OH radical adduct was buried in measurement noise and was not able to be confirmed in the ESR spectrum, it was determined that OH radicals were not present, "Not present" was described for the OH radicals in Table 4, which will be shown later.

Examples of the present disclosure will be further described. Unless otherwise particularly specified, operations and evaluation methods similar to those in Example 1 were conducted.

Example 2

An ultrasonic vibrator having a resonance frequency of 1.7 MHz (trade name: PZT ultrasonic mist maker, ceramic atomizing disk KS-20-1.7 manufactured by Gazechimp) was used as the ultrasonic atomizer disposed in the internal space of the producing section 101 in Example 1. In addition, an external power supply was used to operate this ultrasonic vibrator, and an AC voltage of 48 Vpp was applied at 1.7 MHz. Except for these points, the active oxygen treatment was conducted similarly to Example 1.

Examples 3 to 6

The ultraviolet irradiance and the irradiation time, or the irradiation time were set as described in Table 3. The active oxygen treatment was conducted similarly to Example 1 in regard to the other points.

Example 7

Sodium bicarbonate was added as a neutralizer to the ozone UFB-containing liquid transferred from the producing section 101 to the first reservoir section 103 to adjust pH of the ozone UFB-containing liquid to 6.9. The active oxygen treatment was conducted similarly to Example 1 in regard to the other points.

Example 8

A nitric acid was added as a neutralizer to the ozone UFB-containing liquid transferred from the producing section 101 to the first reservoir section 103 to adjust pH of the ozone UFB-containing liquid to 1.2. The active oxygen treatment was conducted similarly to Example 1 in regard to the other points.

Example 9

As the ozone generating device 111, a commercially available ozone generating device (trade name: O3 Clear 3; manufactured by Ozonemart), which was not a PA, was prepared. This ozone generating device was disposed outside a casing of the producing section 101 as illustrated in Fig. 3, a flow rate regulating pump 302 was interposed in a middle of an ozone supply path 301 from the ozone generating device to regulate an amount of ozone to be supplied to the internal space of the producing section 101, and the ozone concentration in the internal space of the producing section 101 was adjusted to be the same as the ozone concentration in Example 1. The active oxygen treatment was conducted similarly to Example 1 in regard to the other points.

For Examples 1 to 9, active oxygen treatment conditions are summarized in Table 3 below.

Table 3

| Example No. | Raw material liquid | UFB generating device resonance frequency | Ozone generating device | pH of ozone UFB-containing liquid | Ultraviolet irradiance $(mW/cm^2)$ | Ultraviolet irradiation time (seconds) | Ultraviolet irradiation amount (mJ/ml) |
|---|---|---|---|---|---|---|---|
| 1 | Tap water | 108KHz | PA | 4.5 | 2.0 | 20 | 50.7 |
| 2 | Tap water | 1.7MHz | PA | 4.5 | 2.0 | 20 | 50.7 |
| 3 | Tap water | 108KHz | PA | 4.5 | 8.0 | 5 | 50.7 |
| 4 | Tap water | 108KHz | PA | 4.5 | 0.2 | 200 | 50.7 |
| 5 | Tap water | 108KHz | PA | 4.5 | 2.0 | 4 | 10.2 |
| 6 | Tap water | 108KHz | PA | 4.5 | 2.0 | 200 | 508.7 |
| 7 | Tap water | 108KHz | PA | 6.9 | 2.0 | 20 | 50.7 |
| 8 | Tap water | 108KHz | PA | 1.2 | 2.0 | 20 | 50.7 |
| 9 | Tap water | 108KHz | 03 Clear | 4.5 | 2.0 | 20 | 50.7 |

The evaluation results of each example are shown in Table 4.

Table 4

| Example No. | Evaluation 1 | Evaluation 2-1 | Evaluation 2-2 | Evaluation 2-3 | Evaluation 3-1 | Evaluation 3-2 | Evaluation 4 | | Evaluation 5 | |
|---|---|---|---|---|---|---|---|---|---|---|
| | Amount of ozone generated by PA ($\mu$g/min) | Ozone concentration in ozone UFB-containing liquid (mg/L) | pH of ozone UFB-containing liquid | Number average air bubble diameter of UFBs in ozone UFB-containing liquid (nm) | Amount of UFBs in ozone UFB-containing liquid (bubbles/mL) | Amount of UFBs in active oxygen UFB-containing liquid (bubbles/mL) | Whether or not active oxygen has been generated | OH radical signal ratio | Number D of E. coli | Decrement digit |
| 1 | 96 | 2.5 | 4.5 | 110 | 3.1E+08 | 3.1E+08 | Generated | 2.52 | Equal to or less than 1.8E + 02 | Equal to or greater than 5.3 |
| 2 | 96 | 2.5 | 4.5 | 100 | 2.5E+08 | 2.5E+08 | Generated | 2.15 | 4.8E+02 | 4.9 |
| 3 | 96 | 2.5 | 4.5 | 110 | 3.1E+08 | 2.6E+08 | Generated | 2.14 | 4.9E+02 | 4.9 |
| 4 | 96 | 2.5 | 4.5 | 110 | 3.1E+08 | 3.1E+08 | Generated | 2.09 | 5.6E+02 | 4.8 |
| 5 | 96 | 2.5 | 4.5 | 110 | 3.1E+08 | 3.1E+08 | Generated | 0.50 | 3.7E+04 | 3.0 |
| 6 | 96 | 2.5 | 4.5 | 110 | 3.1E+08 | 3.1E+08 | Generated | 1.71 | 1.5E+03 | 4.4 |
| 7 | 96 | 2.5 | 6.9 | 110 | 3.1E+08 | 3.1E+08 | Generated | 1.89 | 9.6E+02 | 4.6 |
| 8 | 96 | 2.5 | 1.2 | 110 | 2.9E+08 | 2.9E+08 | Generated | 2.39 | 2.6E+02 | 5.2 |
| 9 | - | 1.2 | 4.5 | 110 | 2.0E+08 | 2.0E+08 | Generated | 0.62 | 2.6E+04 | 3.1 |

In the table, description of "3.1E + 08", for example, indicates "3.1 $\times 10^8$". The amount of UFBs in the active oxygen UFB-containing liquid is the amount of UFBs in the active oxygen UFB-containing liquid used for the sanitization treatment test. In regard to whether or not active oxygen has been generated, results of the above-described ESR analysis are shown. D is the number of remaining Escherichia coli in the treatment of the active oxygen UFB-containing liquid. The decrement digit is the decrement digit of the number of bacteria obtained by the sanitization treatment.

Here, in comparison between Example 1 and Example 9, the ozone concentration in the ozone UFB-containing liquid in Example 1 was equal to or greater than double the ozone concentration in Example 9 as shown in Table 4 regardless of the fact that the same ozone concentration was set in the internal space of the producing section 101. This is considered to mean that the ozone was more efficiently taken into the UFBs by using the PA as the ozone generating device.

Comparative Example 1

Energy application (ultraviolet irradiation) to the ozone UFB-containing liquid stored in the first reservoir section 103 was not performed. The substance to be treated was treated similarly to Example 1 in regard to the other points.

Comparative Example 2

A treatment device 1201 having the configuration illustrated in Fig. 12 was prepared. In other words, the configuration in which the active oxygen supply device 105 was attached on the side opposite to the side on which the valve 102 of the reservoir section 103 was provided in the active oxygen treatment device according to Example 9 and the ozone UFB-containing liquid stored in the internal space of the reservoir section 103 was able to be atomized to the substance to be treated was adopted. An ultraviolet lamp 108 was disposed such that the mist of the ozone UFB-containing liquid atomized from the active oxygen supply device 105 was able to be irradiated with ultraviolet rays. As the active oxygen treatment device 105, the ultrasonic atomizer that was the same as the ultrasonic atomizer used for the active oxygen UFB-containing liquid supply device of the active oxygen treatment device according to Example 1 was used.

Then, the ozone UFB-containing liquid was produced similarly to Example 9, and the ozone UFB-containing liquid stored in the internal space of the reservoir section 103 was atomized by the ultrasonic atomizer such that the ozone UFB-containing liquid passed through the region irradiated with ultraviolet rays. The mist having passed through the ultraviolet irradiation region was collected and used for evaluation. The results are shown in Table 4. In other words, although the collected liquid included UFBs, it was not possible to confirm presence of active oxygen. Also, the sanitization effect thereof was also inferior to the sanitization effect in each example. Thus, it was considered to be not possible to change the ozone in the UFBs to active oxygen even by irradiating the mist of the ozone UFB-containing liquid with ultraviolet rays in this comparative example.

Comparative Example 3

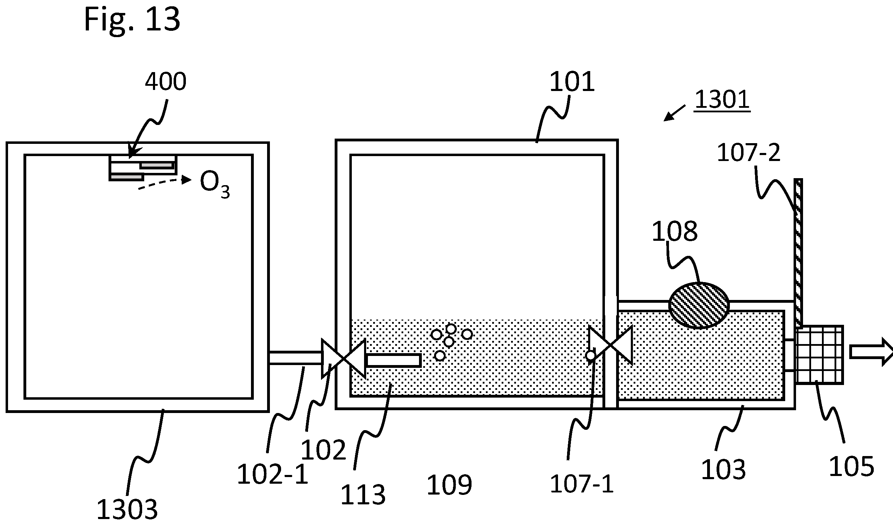

A treatment device having the configuration illustrated in Fig. 13 was prepared. In other words, an ozone generating section 1303 incorporating the PA prepared in Example 1 was prepared. The ozone generating section 1303 was made of an ABS resin in a size of 135 mm in depth $\times$ 30 mm in width $\times$ 30 mm in height. The ozone generated in the ozone generating section 1303 was able to be transferred to the internal space of the producing section 101 by a pump, which is not illustrated, through the supply path 102-1.

Furthermore, 60 mL of tap water was stored in the internal space of the producing section 101 such that the water surface was positioned further upward than the upper end of the opening of the supply path 102-1 of air including ozone from the ozone generating section 1303 on the side of the producing section 101 by 1 cm.

Then, the PA 400 was operated under conditions similar to those in Example 1 in a state where the opening/closing device 102 was closed to thereby generate ozone in the internal space of the ozone generating section 1303.

Then, the opening/closing device 102 was opened to cause the air including ozone inside the ozone generating section 1303 to move to the producing section 101 using a pump (not illustrated) and bring the water stored in the producing section 101 into contact with the air including ozone (bubbling). Then, the bubbling was continued until the ozone concentration in water stored in the internal space of the producing section 101 reached 2.5 mg/L.

Immediately after the bubbling was stopped, 15 mL of liquid in the producing section 101 was caused to move to the first reservoir section 103 in a state where the opening/closing device 107-1 was opened and the opening/closing device 107-2 was closed. Thereafter, the opening/closing device 107-1 was closed to achieve the state where the liquid was stored in the first reservoir section. Then, the liquid stored in the first reservoir section was irradiated with ultraviolet rays under the same conditions as those in Example 1.

Then, the liquid having undergone the ultraviolet irradiation treatment was supplied to the substance to be treated similarly to Example 1.

The results of Evaluation 1 to Evaluation 5 of this comparative example are shown in Table 5.

As shown in Table 5, UFB formation and active oxygen generation were not observed in this comparative example, and a sanitization effect was also not obtained. It is considered that UFBs were not substantially formed and only unstable coarse bubbles were formed when the water was bubbled with air including ozone. In other words, it is considered that bubbles including ozone were not able to be stably present in the water stored in the producing section 101 after the bubbling ended and the ozone taken into the water was immediately released as gas. Therefore, it is considered that the water as the target of irradiation includes substantially no ozone and no active oxygen was generated even through the irradiation with the ultraviolet rays in the first reservoir section.

Comparative Example 4

A UFB-containing liquid that did not include ozone was produced, and a device capable of emitting ultraviolet rays with two peaks at a wavelength of 185 nm and a wavelength of 254 nm was disposed on the upper side with a distance from the liquid surface of the UFB-containing liquid, and irradiation was performed. Whether or not the ozone UFB-containing liquid was generated and whether or not the active oxygen UFB-containing liquid was generated were confirmed. Specific description will be given below.

A UFB-containing liquid that did not include ozone was prepared by not operating the PA of the ozone UFB-containing liquid producing section and by operating the ultrasonic vibrator in Example 1.

Also, the ultraviolet lamp disposed in the first reservoir section was changed to an ultraviolet lamp (cold-cathode tube ultraviolet lamp, trade name: UW/9E89/9, manufactured by Stanley Electric Co., Ltd., a cylindrical shape with a diameter of 9 mm, a peak wavelength = 185 nm + 254 nm) having the same shape as that of the ultraviolet lamp used in Example 1 and having peak wavelengths at 185 nm and 254 nm. A stable ultraviolet irradiatable part of the ultraviolet lamp was a longitudinal part of 135 mm in the cylindrical part.

After it was confirmed that the amount of UFBs in the ozone UFB-containing liquid generated by the producing section 101 was $3.1 \times 10^8$ bubbles/ml, 15 mL of the ozone UFB-containing liquid was transferred to the first reservoir section. Then, the UFB-containing liquid stored in the first reservoir section 103 was irradiated with ultraviolet rays for 2 minutes by the ultraviolet lamp described above. Evaluations 1 to 5 were performed similarly to Example 1 in regard to the other points. The results are shown in Table 5.

As shown in Table 5, although ozone UFB generation was observed, no active oxygen generation was observed, and sanitization performance was significantly lower than the sanitization performance achieved in the examples. The sanitization effect of this comparative example was presumed to be caused by the ozone generated in the liquid through the ultraviolet irradiation in the first reservoir section.

In this comparative example, the reason why the generation of active oxygen was not observed even through the ultraviolet irradiation is considered to be because ozone was not taken in the preparation of the UFB-containing liquid and only air was included in the UFBs.

Table 5

| Comparative Example | Evaluation 1 | Evaluation 2-1 | Evaluation 2-2 | Evaluation 2-3 | Evaluation 3-1 | Evaluation 3-2 | Evaluation 4 | | Evaluation 5 | |
|---|---|---|---|---|---|---|---|---|---|---|
| | Amount of ozone generated by PA ($\mu$ g/min) | Ozone concentration in ozone UFB-containing liquid (mg/L) | pH of ozone UFB-containing liquid | Number average air bubble diameter of UFBs in ozone UFB-containing liquid (nm) | Amount of UFBs in ozone UFB-containing liquid (bubbles/mL) | Amount of UFBs in active oxygen UFB-containing liquid (bubbles/mL) | Whether or not active oxygen has been generated | OH radical signal ratio | Number D of E. coli | Decrement digit |
| 1 | 96 | 2.5 | 4.5 | 110 | 3.1E+08 | 3.1E+08 | Not generated | - | 3.7E+06 | 1.0 |
| 2 | 96 | 2.5 | 4.5 | 110 | 3.1E+08 | 3.1E+08 | Not generated | - | 7.3E+06 | 0.7 |
| 3 | 96 | 2.5 | 4.5 | - | Not detected | Not detected | Not generated | - | 2.3E+07 | 0.2 |
| 4 | - | - | 6.0 | - | 3.1E+08 | 3.1E+08 | Not generated | - | 4.6E+06 | 0.9 |

The present disclosure is not limited to the above embodiments, and various changes and modifications are possible without departing from the spirit and scope of the present disclosure. Therefore, the following claims are appended to make the scope of the present disclosure public.

This application claims the benefit of Japanese Patent Application No. 2023-138429, filed August 28, 2023, which is hereby incorporated by reference herein in its entirety.

## Claims

1.  An active oxygen treatment device comprising:

    an ozone UFB-containing liquid producing section that produces an ozone UFB-containing liquid in which ultrafine bubbles comprising ozone are comprised in a liquid;
    a first reservoir section that stores the ozone UFB-containing liquid;
    an energy application device that applies energy necessary to decompose at least a part of the ozone comprised in the ultrafine bubbles into active oxygen to the ozone UFB-containing liquid in a stored state in the first reservoir section and decomposes at least a part of the ozone into active oxygen; and
    an active oxygen UFB-containing liquid supply device that supplies, to a substance to be treated, an active oxygen UFB-containing liquid that has been generated in the first reservoir section by decomposing at least a part of the ozone in the ultrafine bubbles into the active oxygen through the application of the energy, the ultrafine bubbles comprising active oxygen being comprised in a liquid in the active oxygen UFB-containing liquid.

2.  The active oxygen treatment device according to claim 1, further comprising:

    a second reservoir section,
    wherein the second reservoir section is configured to be capable of receiving the active oxygen UFB-containing liquid generated in the first reservoir section.

3.  The active oxygen treatment device according to claim 2, wherein the active oxygen UFB-containing liquid supply device is configured to be capable of supplying the active oxygen UFB-containing liquid stored in the second reservoir section to a substance to be treated.

4.  The active oxygen treatment device according to any one of claims 1 to 3, wherein the active oxygen UFB-containing liquid supply device comprises an atomizing device that atomizes the active oxygen UFB-containing liquid to the substance to be treated.

5.  The active oxygen treatment device according to any one of claims 1 to 4, wherein content of the ultrafine bubbles with bubble diameters of equal to or less than 500 nm per unit volume in the ozone UFB-containing liquid is from $1.0 \times 10^6$ to $1.0 \times 10^{10}$ bubbles/mL.

6.  The active oxygen treatment device according to any one of claims 1 to 5, further comprising:

    an ozone generating device that supplies the ozone to the ozone UFB-containing liquid producing section, wherein the ozone generating device is a plasma actuator, and
    the plasma actuator is provided with a first electrode and a second electrode with a dielectric body sandwiched therebetween and generates an induced flow comprising the ozone by applying a voltage between the first electrode and the second electrode.

7.  The active oxygen treatment device according to any one of claims 1 to 6, wherein the energy application device is an ultraviolet irradiation device comprising an ultraviolet light source.

8.  A treatment method with active oxygen, comprising:

    (1) producing an ozone UFB-containing liquid in which ultrafine bubbles comprising ozone are comprised in a liquid;
    (2) storing the produced ozone UFB-containing liquid in a first reservoir section;
    (3) applying energy necessary to decompose at least a part of the ozone comprised in the ultrafine bubbles into active oxygen to the ozone UFB-containing liquid in a stored state in the first reservoir section, decomposing at least part of the ozone into the active oxygen, and preparing an active oxygen UFB-containing liquid in which the ultrafine bubbles comprising the active oxygen are comprised in a liquid; and

(4) supplying the active oxygen UFB-containing liquid stored in the first reservoir section to a substance to be treated.

9. The treatment method with active oxygen according to claim 8, wherein in (3), the energy is applied to the ozone UFB-containing liquid in the first reservoir section without the ozone UFB-containing liquid flowing into the first reservoir section and the ozone UFB-containing liquid flowing out of the first reservoir section.

10. An ozone UFB-containing liquid producing device that generates an ozone UFB-containing liquid in which ultrafine bubbles comprising ozone are comprised in a liquid, the ozone UFB-containing liquid generating device comprising:

a liquid reservoir section that stores a liquid,
wherein the liquid reservoir section comprises
a UFB generating device that produces a UFB-containing liquid in which the ultrafine bubbles are comprised in the liquid stored in the liquid reservoir section, and
a plasma actuator,
the plasma actuator is provided with a first electrode and a second electrode with a dielectric body sandwiched therebetween and generates an induced flow comprising the ozone by applying a voltage between the first electrode and the second electrode, and
the plasma actuator is disposed such that the induced flow flows inside the liquid reservoir section.

11. The ozone UFB-containing liquid producing device according to claim 10, wherein the UFB generating device comprises an ultrasonic vibrator that causes the liquid to comprise the ultrafine bubbles and atomizes the liquid.

12. The ozone UFB-containing liquid producing device according to claim 10 or 11, wherein when the plasma actuator is installed and operated in a container, gas inside the container is collected, and an ozone concentration (ppm) is measured, an amount of generated ozone per unit time calculated by Equation (A) below is equal to or greater than 15 µg/min.

Equation (A)

amount of generated ozone per unit time (µg/min)

= measured ozone concentration (ppm) × molecular weight of ozone/22.4 × 273/(273

+ room temperature (°C)) × gas volume (L) inside container

**Amended claims under Art. 19.1 PCT**

1. (Amended) An active oxygen treatment apparatus comprising:

an ozone UFB-containing liquid producing section that produces an ozone UFB-containing liquid in which ultrafine bubbles comprising ozone are comprised in a liquid;
a first reservoir section that stores the ozone UFB-containing liquid;
an energy application device that applies energy necessary to decompose at least a part of the ozone comprised in the ultrafine bubbles into active oxygen to the ozone UFB-containing liquid in a stored state in the first reservoir section and decomposes at least a part of the ozone into active oxygen; and
an active oxygen UFB-containing liquid supply device that supplies, to a substance to be treated, an active oxygen UFB-containing liquid that has been generated in the first reservoir section by decomposing at least a part of the ozone in the ultrafine bubbles into the active oxygen through the application of the energy, the ultrafine bubbles comprising active oxygen being comprised in a liquid in the active oxygen UFB-containing liquid,

wherein

the stored state refers to a state without the ozone UFB-containing liquid flowing into the first reservoir section and the ozone UFB-containing liquid flowing out of the first reservoir section, and

the energy application device is an ultraviolet irradiation device comprising an ultraviolet light source.

2. The active oxygen treatment apparatus according to claim 1, further comprising:

a second reservoir section,
wherein the second reservoir section is configured to be capable of receiving the active oxygen UFB-containing liquid generated in the first reservoir section.

3. The active oxygen treatment apparatus according to claim 2, wherein the active oxygen UFB-containing liquid supply device is configured to be capable of supplying the active oxygen UFB-containing liquid stored in the second reservoir section to a substance to be treated.

4. The active oxygen treatment apparatus according to any one of claims 1 to 3, wherein the active oxygen UFB-containing liquid supply device comprises an atomizing device that atomizes the active oxygen UFB-containing liquid to the substance to be treated.

5. The active oxygen treatment apparatus according to any one of claims 1 to 4, wherein content of the ultrafine bubbles with bubble diameters of equal to or less than 500 nm per unit volume in the ozone UFB-containing liquid is from $1.0 \times 10^6$ to $1.0 \times 10^{10}$ bubbles/mL.

6. The active oxygen treatment apparatus according to any one of claims 1 to 5, further comprising:

an ozone generating device that supplies the ozone to the ozone UFB-containing liquid producing section,
wherein the ozone generating device is a plasma actuator, and
the plasma actuator is provided with a first electrode and a second electrode with a dielectric body sandwiched therebetween and generates an induced flow comprising the ozone by applying a voltage between the first electrode and the second electrode.

7. (Cancelled)

8. (Amended) A treatment method with active oxygen, comprising:

(1) producing an ozone UFB-containing liquid in which ultrafine bubbles comprising ozone are comprised in a liquid;
(2) storing the produced ozone UFB-containing liquid in a first reservoir section;
(3) applying energy necessary to decompose at least a part of the ozone comprised in the ultrafine bubbles into active oxygen to the ozone UFB-containing liquid in a stored state in the first reservoir section, decomposing at least part of the ozone into the active oxygen, and preparing an active oxygen UFB-containing liquid in which the ultrafine bubbles comprising the active oxygen are comprised in a liquid; and
(4) supplying the active oxygen UFB-containing liquid stored in the first reservoir section to a substance to be treated, wherein
the stored state refers to a state without the ozone UFB-containing liquid flowing into the first reservoir section and the ozone UFB-containing liquid flowing out of the first reservoir section, and
the energy application is irradiation with ultraviolet rays.

9. (Cancelled)

10. An ozone UFB-containing liquid producing apparatus that generates an ozone UFB-containing liquid in which ultrafine bubbles comprising ozone are comprised in a liquid, the ozone UFB-containing liquid generating apparatus comprising:

a liquid reservoir section that stores a liquid,
wherein the liquid reservoir section comprises
a UFB generating device that produces a UFB-containing liquid in which the ultrafine bubbles are comprised in the liquid stored in the liquid reservoir section, and
a plasma actuator,
the plasma actuator is provided with a first electrode and a second electrode with a dielectric body sandwiched therebetween and generates an induced flow comprising the ozone by applying a voltage between the first electrode and the second electrode, and
the plasma actuator is disposed such that the induced flow flows inside the liquid reservoir section.

11. The ozone UFB-containing liquid producing apparatus according to claim 10, wherein the UFB generating device comprises an ultrasonic vibrator that causes the liquid to comprise the ultrafine bubbles and atomizes the liquid.

12. The ozone UFB-containing liquid producing apparatus according to claim 10 or 11, wherein when the plasma actuator is installed and operated in a container, gas inside the container is collected, and an ozone concentration (ppm) is measured, an amount of generated ozone per unit time calculated by Equation (A) below is equal to or greater than 15 μg/min.

Equation (A)

amount of generated ozone per unit time (μg/min)

= measured ozone concentration (ppm) × molecular weight of ozone/22.4 × 273/(273 +

room temperature (°C)) × gas volume (L) inside container

Fig. 1

100

104

102    101    107-1    103    107-2    105

Fig. 2A

Fig. 2B

Fig. 3

Fig. 4A

400

Fig. 4B

## Fig. 5

Fig. 6A

Fig. 6B

Fig. 7

Fig. 8

Fig. 9

Fig. 10A

Fig. 10B

## Fig. 11

1102
1101

## Fig. 12

111

1201

301  302

101

107-2

O₃

108

UFB-containing mist

102-1
102

113    109

105

Fig. 13

1301

107-2

105

108

103

101

107-1

109

102

102-1

113

400

O₃

1303

## Fig. 14

EP 4 772 200 A1

## INTERNATIONAL SEARCH REPORT

| International application No. |
|---|
| **PCT/JP2024/030128** |

**A. CLASSIFICATION OF SUBJECT MATTER**

*A61L 2/18*(2006.01)i; *H05H 1/24*(2006.01)i; *A61L 101/10*(2006.01)n
FI:   A61L2/18; H05H1/24; A61L101:10

According to International Patent Classification (IPC) or to both national classification and IPC

**B. FIELDS SEARCHED**

Minimum documentation searched (classification system followed by classification symbols)

A61L2/18; H05H1/24; A61L101/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2022-64317 A (TOSSLEC KK) 25 April 2022 (2022-04-25) paragraphs [0029]-[0098], fig. 1-4 | 1-3, 8-9 |
| Y | paragraphs [0029]-[0098], fig. 1-4 | 4-7, 10-12 |
| Y | JP 2022-36815 A (MIIKE IRON WORKS CO., LTD.) 08 March 2022 (2022-03-08) paragraphs [0037]-[0072] | 4-7, 10-12 |
| Y | JP 2022-13730 A (CANON KABUSHIKI KAISHA) 18 January 2022 (2022-01-18) paragraphs [0020]-[0021] | 6-7, 10-12 |
| Y | JP 2006-280341 A (KURASHIKI BOSEKI K.K.) 19 October 2006 (2006-10-19) paragraphs [0018]-[0081] | 7 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **30 September 2024** | **08 October 2024** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

INTERNATIONAL SEARCH REPORT
Information on patent family members

International application No.

**PCT/JP2024/030128**

| Patent document cited in search report | | | Publication date (day/month/year) | Patent family member(s) | Publication date (day/month/year) |
|---|---|---|---|---|---|
| JP | 2022-64317 | A | 25 April 2022 | (Family: none) | |
| JP | 2022-36815 | A | 08 March 2022 | (Family: none) | |
| JP | 2022-13730 | A | 18 January 2022 | US 2023/0139536 A1 paragraphs [0056]-[0067] US 2023/0146111 A1 WO 2022/004763 A1 WO 2022/004771 A1 EP 4173648 A1 EP 4174019 A1 KR 10-2023-0011424 A CN 115996762 A KR 10-2023-0013108 A CN 115997482 A | |
| JP | 2006-280341 | A | 19 October 2006 | (Family: none) | |

Form PCT/ISA/210 (patent family annex) (July 2022)

**REFERENCES CITED IN THE DESCRIPTION**

**Patent documents cited in the description**

- JP 2022036815 A
- JP 2023058216 A
- JP 2023138429 A